(19)

Europäisches Patentamt
European Patent Office
Office européen des brevets

(11) **EP 2 950 712 B1**

(12) **EUROPEAN PATENT SPECIFICATION**

(45) Date of publication and mention
of the grant of the patent:
**28.10.2020 Bulletin 2020/44**

(51) Int Cl.:
*A61B 5/00* (2006.01)   *A61B 5/06* (2006.01)
*A61B 5/103* (2006.01)   *A61B 8/00* (2006.01)
*A61B 8/08* (2006.01)   *A61B 17/17* (2006.01)
*G01S 5/16* (2006.01)   *G01S 15/89* (2006.01)
*A61B 90/00* (2016.01)

(21) Application number: **14746059.6**

(22) Date of filing: **04.02.2014**

(86) International application number:
**PCT/US2014/014526**

(87) International publication number:
**WO 2014/121244 (07.08.2014 Gazette 2014/32)**

(54) **SYSTEM FOR 3D RECONSTRUCTION OF A JOINT USING ULTRASOUND**

SYSTEM ZUR 3D-REKONSTRUKTION EINER VERBINDUNG MIT ULTRASCHALL

SYSTÈME POUR RECONSTRUCTION TRIDIMENSIONNELLE (3D) D'UNE ARTICULATION
UTILISANT D'ULTRASONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.02.2013 US 201313758151**

(43) Date of publication of application:
**09.12.2015 Bulletin 2015/50**

(73) Proprietor: **Jointvue, LLC
Columbus, OH 43212-1155 (US)**

(72) Inventors:
• **MAHFOUZ, Mohamed, R.
Knoxville, TN 37922 (US)**
• **WASIELEWSKI, Ray, C.
New Albany, OH 43054 (US)**

(74) Representative: **Hutchison, Craig McGregor
Lawrie IP Limited
310 St Vincent Street
Glasgow G2 5RG (GB)**

(56) References cited:
WO-A1-2012/018851    JP-A- 2005 095 320
US-A1- 2005 240 098    US-A1- 2009 129 652
US-A1- 2010 256 504    US-A1- 2011 054 313
US-A1- 2011 125 016    US-A1- 2012 128 223
US-A1- 2012 277 588

• **CALVIN R MAURER ET AL: "AcouStick: A
Tracked A-Mode Ultrasonography System for
Registration in Image-Guided Surgery", 1
January 2006 (2006-01-01), MEDICAL IMAGE
COMPUTING AND COMPUTER ASSISTED
INTERVENTION - MICCAI '99 : SECOND
INTERNATIONAL CONFERENCE, CAMBRIDGE,
UK, SEPTEMBER 19 - 22, 1999; [LECTURE NOTES
IN COMPUTER SCIENCE ; 1679], SPRINGER,
BERLIN [U.A.], PAGE(S) 953 - 962, XP019036122,
ISBN: 978-3-540-66503-8 * page 954, paragraph 1
- page 956, paragraph 2; figures 1,2 ***
• **DEMIRLI R ET AL: "An efficient sparse signal
decomposition technique for ultrasonic signal
analysis using envelope and instantaneous
phase", ULTRASONICS SYMPOSIUM, 2008. IUS
2008. IEEE, IEEE, PISCATAWAY, NJ, USA, 2
November 2008 (2008-11-02), pages 1503-1507,
XP031443650, ISBN: 978-1-4244-2428-3**
• **Zhe Chen ET AL: "Bayesian Filtering: From
Kalman Filters to Particle Filters, and Beyond", ,
1 January 2003 (2003-01-01), XP055242498,
Retrieved from the Internet:
URL:http://www.dsi.unifi.it/users/chisci/i
dfric/Nonlinear_filtering_Chen.pdf [retrieved on
2016-01-18]**

**Description**

**TECHNICAL FIELD**

The present invention relates generally to methods of generating 3-D models of musculoskeletal systems and, more particularly, to ultrasound based 3-D bone and cartilage model reconstruction.

**BACKGROUND**

The reconstruction of a 3-D model for joint, such as the articulating bones of a knee, is a key component of computer-aided joint surgery systems. The existence of a pre-operatively acquired model enables the surgeon to pre-plan a surgery by choosing the proper implant size, such as calculating the femoral and tibial cutting planes in the case of knee surgery, and evaluating the fit of the chosen implant. The conventional method of generating the 3-D model is segmentation of computed tomography ("CT"), or magnetic resonance imaging ("MRI") scans, which are the conventional imaging modalities for creating patient-specific 3-D bone models. The segmentation methods used are either manually, semi-automatic, or fully automated. Although these methods produce highly accurate models, CT and MRI have inherent draw backs, i.e., both are fairly expensive procedures (especially for the MRI), and CT exposes the patient to ionizing radiation.

One alternative method of forming patient-specific models is the use of previously acquired X-Ray images as *a priori* information to guide the morphing of a template bone model whose projection matches the X-Ray images. Several X-Ray based model reconstruction methodologies have been developed for the femur (including, specifically, the proximal and distal portions), the pelvis, the spine, and the rib cage.

Conventional ultrasound imaging utilizes B-mode images. B-mode images are constructed by extracting an envelope of received scanned lines of radiofrequency ("RF") signals using the Hilbert transformation. These envelopes are then decimated (causing a drop in the resolution) and converted to grayscale (usually 256 bit) to form the final B-mode image. The conversion to grayscale results in a drop in the dynamic range of the ultrasound data.

WO2012/018851 discloses a method of generating a 3-D patient-specific musculoskeletal model. The method includes acquiring a plurality of raw radiofrequency ("RF") signals from an A-mode ultrasound scan of the bone, while tracking the acquiring probe in 3D space. The bone contours are isolated in each of the plurality of RF signals and transformed into a point cloud. A 3-D bone model of the bone (116, 118, 120) is then optimized with respect to the point cloud.

The use of ultrasound in computer aided orthopedic surgery has gained interest in the recent decade due to its relatively low cost and radiation-free nature. More particularly, A-mode ultrasound intra-operative registration has been used for computer aided orthopedic surgery and, in limited cases, in neurosurgery. Ultrasound-MRI registration has been developed utilizing B-mode ultrasound images. However, it has proven difficult to generate 3-D bone models having sufficient quality using conventional ultrasound technology due to limitations in the quality of the images.

Therefore, there is a need to develop improved apparatuses and methods that utilized ultrasound techniques to construct 3-D patient-specific bone and cartilage models.

**SUMMARY**

The invention is defined in the independent claims 1 and 16.

The present invention overcomes the foregoing problems and other shortcomings, drawbacks, and challenges of high cost or high radiation exposure imaging modalities to generate a patient-specific model by ultrasound techniques. While the present invention will be described in connection with certain embodiments, it will be understood that the present invention is not limited to these embodiments, but to the appended claims.

In accordance with one embodiment of the present invention, a method of generating a 3-D patient-specific bone model is described. The method includes acquiring a plurality of raw radiofrequency ("RF") signals from an A-mode ultrasound scan of the bone, which is spatially tracked in 3-D space. The bone contours are isolated in each of the plurality of RF signals and transformed into a point cloud. A 3-D model of the bone is then optimized with respect to the point cloud.

According to another embodiment of the present invention, a method for 3-D reconstruction of a bone surface includes imaging the bone with A-mode ultrasound. A plurality of RF signals is acquired while imaging. Imaging of the bone is also tracked. A bone contour is extracted from each of the plurality of RF signals. Then, using the tracked data and the extracted bone contours, a point cloud representing the surface of the bone is generated. A model of the bone is morphed to match the surface of the bone as represented by the point cloud.

In yet another embodiment of the present invention, a computer method for simulating a surface of a bone is described. The computer method includes executing a computer program in accordance with a process. The process includes extracting a bone contour from each of a plurality of A-mode RF signals. The extracted bone contours are transformed from a local frame of reference into a point cloud in a world-frame of reference. A generalized model of the bone is compared with the point cloud and, as determined from the comparing, the generalized model is deformed to match the point cloud.

Another embodiment of the present invention is directed to a computer program product that includes a non-transitory computer readable medium and program instructions stored on the computer readable medium. The program instructions, when executed by a process, cause the computer program product to isolate a bone contour from a plurality of RF signals. The plurality of RF signals being previously acquired from a reflected A-mode ultrasound beam. The bone contours are then transformed into a point cloud and used to optimize a 3-D model of the bone.

Still another embodiment of the present invention is directed to a computing device having a processor and a memory. The memory includes instructions that, when executed by the processor, cause the processor to isolate a bone contour from a plurality of RF signals. The plurality of RF signals being previously acquired from a reflected A-mode ultrasound beam. The bone contours are then transformed into a point cloud and used to optimize a 3-D model of the bone.

## BRIEF DESCRIPTION OF THE FIGURES

The accompanying drawings, which are incorporated in and constitute a part of this specification, illustrate embodiments of the present invention and, together with the detailed description of the embodiments given below, serve to explain the principles of the present invention.

FIG. 1 is a perspective view of an ultrasound instrument in accordance with one embodiment of the present invention.
FIG. 2 is a perspective view of a hybrid probe comprising an ultrasound probe and an optical marker, in accordance with one embodiment of the present invention.
FIG. 2A is a side elevational view of a position sensor for use with the optical marker of the hybrid probe.
FIG. 3 is a diagrammatic view of a computer system suitable for generating a 3-D patient-specific bone model from A-mode ultrasound RF signals in accordance with one embodiment of the present invention.
FIG. 4 is a flow chart illustrating one exemplary method of calibrating the optical system and generating a transformation between a local frame and a world frame.
FIGS. 5A-5C are diagrammatic views of a knee joint, showing the anterior, the medial, and the posterior portions, respectively.
FIGS. 6A-6F are fluoroscopic images of the knee joint in a plurality of degrees of flexion.
FIG. 7 is a flow chart illustrating one exemplary method of acquiring A-mode ultrasound RF signal and generating the 3-D patient-specific bone model.
FIG. 8 is a diagrammatic view of the method of acquiring A-mode ultrasound RF signals in accordance with FIG. 7.
FIG. 9 is a B-mode ultrasound image of a knee joint, which may be generated from the A-mode ultrasound RF signal.
FIG. 10A is an example of a raw RF signal as acquired by one transducer of the transducer array of an ultrasound probe.
FIG. 10B is the ultrasound frame illustrates select ones of the RF signals overlaid the B-mode ultrasound image of FIG. 9.
FIG. 10C is the ultrasound frame of FIG. 9B with a bone echo contour identified.
FIG. 10D is a 3-D rendering of the RF signals acquired in a data frame, which is shown in the B-mode image format in FIG. 10C.
FIG. 10E is another 3-D rendering of an ultrasound frame with select ones of the RF signals delineated.
FIG. 11 is a flow chart illustrating one exemplary method of identifying and extracting the bone echo from the A-mode ultrasound RF signal.
FIG. 12A is a 3-D rendering of an ultrasound frame after envelope detection.
FIGS. 12B-12E respectively illustrate four exemplary envelopes of the sampled A-mode ultrasound RF signal, with the echoes identified in each envelope.
FIGS. 13A and 13D are B-mode ultrasound frames calculated from exemplary A-mode ultrasound RF signals.
FIGS. 13B and 13E are ultrasound frames corresponding to FIGS. 13A and 13-D, respectively, with a bone contour identified before noise removal and overlain on the B-mode image.
FIGS. 13C and 13F are plots of the local standard deviation of the bone contours of FIGS. 13B and 13E, respectively.
FIGS. 14A and 14D are ultrasound frames illustrating exemplary B-mode images constructed from A-mode ultrasound RF signals, and in which no bone tissue was scanned.
FIGS. 14B and 14E are ultrasound frames corresponding to FIGS. 14A and 14D, respectively, with the noisy false bone contours shown.
FIGS. 14C and 14F are plots of the local standard deviation of the last echoes of FIGS. 14B and 14E, respectively.
FIG. 15 is a flow chart illustrating one exemplary method of generating a bone point cloud from the isolated bone

EP 2 950 712 B1

contours.

FIGS. 16A, 16C, 17A, and 17C are exemplary bone point clouds, generated in accordance with one embodiment of the present invention.

FIGS. 16B, 16D, 17B, and 17D are examples in which the bone point clouds of FIGS. 16A, 16C, 17A, and 17C, respectively, are aligned to a bone model.

FIG. 18 is a flow chart illustrating one exemplary method of generating a statistical atlas of bone models.

FIG. 19 is a flow chart illustrating one exemplary method of optimizing a bone model to the bone point cloud.

FIG. 20 is a diagrammatic view of a medical imaging system including an ultrasound machine, electromagnetic tracking system, and a computer that operate cooperatively to provide real-time 3-D images to the attending physician.

FIG. 21 is a flow chart illustrating a method in accordance with an alternative embodiment of the invention by which the imaging system in FIG. 20 generates a real-time 3-D bone model.

FIG. 22 is a graphical view illustrating an ultrasound signal that is swept in frequency.

FIGS. 23A and 23B are graphical views illustrating an RF signal, a signal envelope generated from the RF signal, and a plurality of amplitude peaks identified in the signal envelope using a linear Gaussian filter.

FIGS. 24A-24D are graphical views illustrating an RF signal, a signal envelope generated from the RF signal, and a plurality of amplitude peaks identified in the signal envelope using a non-linear, non-Gaussian filter.

FIG. 25 is a graphical view illustrating one method by which a contour line is derived from a plurality of ultrasound scan line signal envelopes.

FIG. 26 is a graphical view illustrating a contour generated from a plurality of ultrasound scan line envelopes using first peak detection, and a contour generated from the plurality of scan line envelopes using a Bayesian smoothing filter.

FIG. 27 is a 3-D view of an ultrasound frame after envelope detection, and a corresponding registered point cloud for an imaged joint.

## DETAILED DESCRIPTION OF THE EMBODIMENTS OF THE INVENTION

The various embodiments of the present invention are directed to methods of generating a 3-D patient-specific bone model. To generate the 3-D patient-specific model, a plurality of raw RF signals is acquired using A-mode ultrasound acquisition methodologies. A bone contour is then isolated in each of the plurality of RF signals and transformed into a point cloud. The point clouds may then be used to optimize a 3-D model of the bone such that the patient-specific model may be generated. Although the various embodiments of the invention are shown herein with respect to a human patient, persons having ordinary skill in the art will understand that embodiments of the invention may also be used to generate 3-D patient-specific bone models of animals (e.g., dogs, horses, etc.) such as for veterinarian applications.

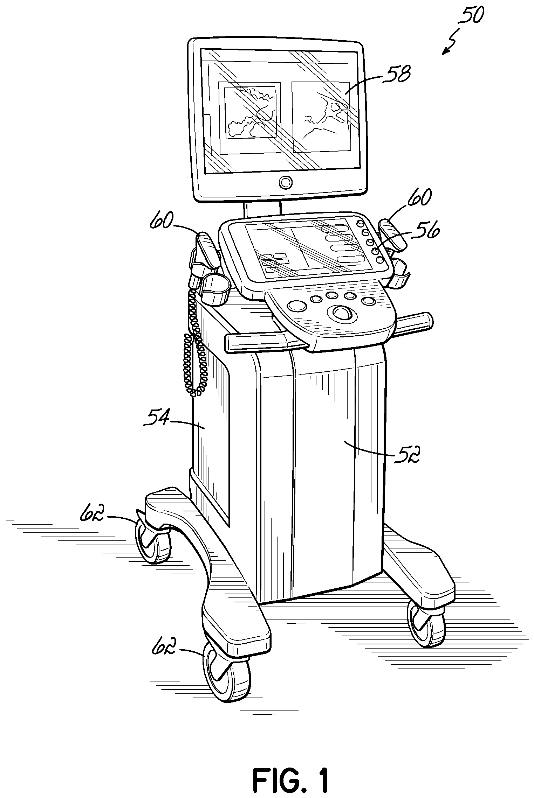

Turning now to the figures, and in particular to FIG. 1, one embodiment of an ultrasound instrument 50 for use with one or more embodiments of the present invention is shown. The ultrasound instrument 50 should be configurable such that the user may access acquired RF ultrasound data. One suitable instrument may, for example, include the diagnostic ultrasound model SonixRP by Ultrasonix Inc. (Richmond, British Columbia, Canada). The ultrasound instrument 50 includes a housing 52 containing a controller, (for example, a computer 54), an energy or power source (not shown), a user input device 56, an output device (for example, a monitor 58), and at least one ultrasound probe 60. The housing 52 may include caster wheels 62 for transporting the ultrasound instrument 50 within the medical facility.

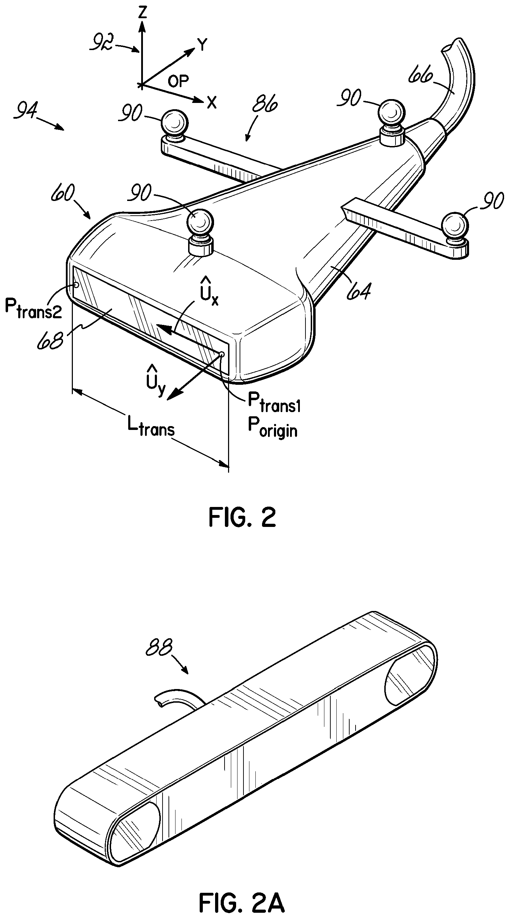

The at least one ultrasound probe 60 is configured to acquire ultrasound raw radiofrequency ("RF") signals, and is shown in greater detail in FIG. 2. The ultrasound probe 60, such as the particular embodiment shown, may be a high resolution linear transducer with a center frequency of 7.5 MHz, as is conventionally used in musculoskeletal procedures. The sampling frequency used in digitizing ultrasound echo may be, for example, 20 MHz and must be at least twice the maximum ultrasound frequency. Generally, the ultrasound probe 60 includes a body 64 that is coupled to the ultrasound instrument housing 52 by a cable 66. The body 64 further includes a transducer array 68 configured to transmit an ultrasound pulse and to receive reflected ultrasound RF energy. The received RF echo is transmitted along the cable 66, to the computer 54 of the ultrasound instrument 50 for processing in accordance with an embodiment of the present invention.

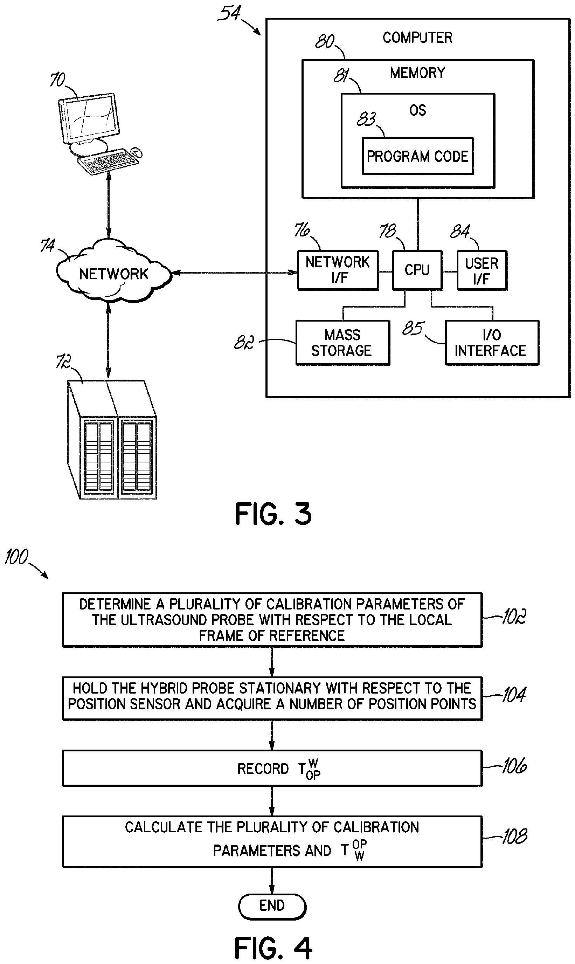

The computer 54 of the ultrasound instrument 50, as shown in FIG. 3, may be considered to represent any type of computer, computer system, computing system, server, disk array, or programmable device such as multi-user computers, single-user computers, handheld devices, networked devices, or embedded devices, etc. The computer 54 may be implemented with one or more networked computers 70 or networked storage devices 72 using one or more networks 74, e.g., in a cluster or other distributed computing system through a network interface 76 (illustrated as "NETWORK I/F"). For brevity's sake, the computer 54 will be referred to simply as "computer," although it should be appreciated that the term "computing system" may also include other suitable programmable electronic devices consistent with embodiments of the present invention.

The computer 54 typically includes at least one processing unit 78 (illustrated as "CPU") coupled to a memory 80 along with several different types of peripheral devices, e.g., a mass storage device 82, the user interface 84 (illustrated as "User I/F," which may include the input device 56 and the monitor 58), the Network I/F 76, and an Input/Output (IO) interface 85 for coupling the computer 54 to additional equipment, such as the aforementioned ultrasound instrument 50. The memory 80 may include dynamic random access memory ("DRAM"), static random access memory ("SRAM"), non-volatile random access memory ("NVRAM"), persistent memory, flash memory, at least one hard disk drive, and/or another digital storage medium. The mass storage device 82 is typically at least one hard disk drive and may be located externally to the computer 54, such as in a separate enclosure or in one or more of the networked computers 70, one or more of the networked storage devices 72 (for example, a server).

The CPU 78 may be, in various embodiments, a single-thread, multi-threaded, multi-core, and/or multi-element processing unit (not shown). In alternative embodiments, the computer 54 may include a plurality of processing units that may include single-thread processing units, multi-threaded processing units, multi-core processing units, multi-element processing units, and/or combinations thereof. Similarly, the memory 80 may include one or more levels of data, instruction, and/or combination caches, with caches serving the individual processing unit or multiple processing units (not shown).

The memory 80 of the computer 54 may include an operating system 81 (illustrated as "OS") to control the primary operation of the computer 54 in a manner known in the art. The memory 80 may also include at least one application, component, algorithm, program, object, module, or sequence of instructions, or even a subset thereof, will be referred to herein as "computer program code" or simply "program code" 83. Program code 83 typically comprises one or more instructions that are resident at various times in the memory 80 and/or the mass storage device 82 of the computer 54, and that, when read and executed by the CPU 78, causes the computer 54 to perform the steps necessary to execute steps or elements embodying the various aspects of the present invention.

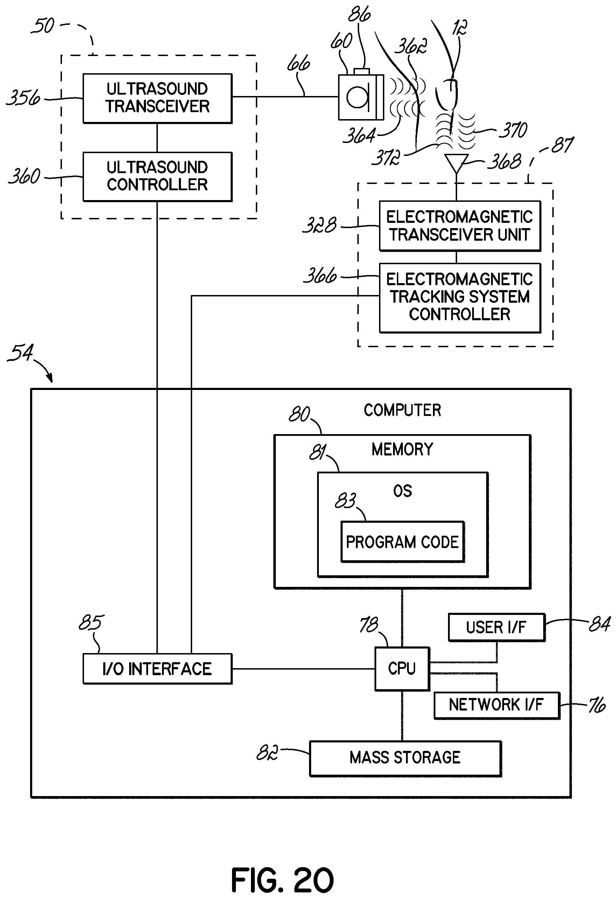

The I/O interface 85 is configured to operatively couple the CPU 78 to other devices and systems, including the ultrasound instrument 50 and an optional electromagnetic tracking system 87 (FIG. 20). The I/O interface 85 may include signal processing circuits that condition incoming and outgoing signals so that the signals are compatible with both the CPU 78 and the components to which the CPU 78 is coupled. To this end, the I/O interface 85 may include conductors, analog-to-digital (A/D) and/or digital-to-analog (D/A) converters, voltage level and/or frequency shifting circuits, optical isolation and/or driver circuits, and/or any other analog or digital circuitry suitable for coupling the CPU 78 to the other devices and systems. For example, the I/O interface 85 may include one or more amplifier circuits to amplify signals received from the ultrasound instrument 50 prior to analysis in the CPU 78.

Those skilled in the art will recognize that the environment illustrated in FIG. 3 is not intended to limit the present invention.

Returning again to FIG. 2, the ultrasound probe 60 has mounted thereto a tracking marker 86, which, for purposes of illustration only, is shown as an optical marker, configured to spatially register the motion of the ultrasound probe 60 during signal acquisition. The tracking marker 86 may be comprised of a plurality of reflective portions 90, which are described in greater detail below. The tracked probe constitutes a hybrid probe 94. In other embodiments, the tracking marker and associated system may be electromagnetic, RF, or any other known 3-D tracking system.

The optical tracking marker 86 is operably coupled to a position sensor 88, one embodiment of which is shown in FIG. 2A. In use, the position sensor 88 emits energy (for example, infrared light) in a direction toward the optical tracking marker 86. Reflective portions 90 of the optical tracking marker 86 reflect the energy back to the position sensor 88, which then triangulates the 3-D position and orientation of the optical tracking marker 86. One example of a suitable optical tracking system is the Polaris model manufactured by Northern Digital Inc. (Waterloo, Ontario, Canada).

The optical tracking marker 86 is rigidly attached to the ultrasound probe 60 and is provided a local coordinate frame of reference ("local frame" 92). Additionally, the ultrasound probe 60 is provided another local coordinate frame of reference ("ultrasound frame"). For the sake of convenience, the combination optical tracking marker 86 with the ultrasound probe 60 is referred to as the "hybrid probe" 94. The position sensor 88, positioned away from the hybrid probe 94, determines a fixed world coordinate frame ("world frame"). Operation of the optical tracking system (the optical tracking marker 86 with the position sensor 88) with the ultrasound probe 60, once calibrated, is configured to determine a transformation between the local and ultrasound coordinate frames.

Turning now to FIG. 4, with continued reference to FIG. 2, a method 100 of calibrating the optical tracking system according to one embodiment of the present invention is described. To calibrate the optical tracking marker 86 with the position sensor 88, for real-time tracking of the hybrid probe 94, a homogeneous transformation $T_{OP}^{W}$ between the local frame, OP, and the world frame, W, is needed. The calibration method 100 begins with determining a plurality of calibration parameters (Block 102). In the particular illustrative example, four parameters are used and include: $P_{trans-origin}$, i.e., a point of origin on the transducer array 68; $L_{trans}$, i.e., a length of the transducer array 68; $\hat{u}_x$, i.e., a unit vector along the length of the transducer array 68; 4) $\hat{u}_y$, i.e., a unit vector in a direction that is perpendicular to the length of the transducer array 68. These calibration points and vectors are relative to the local frame 92 ("OP").

The hybrid probe is held in a fixed position while the position sensor 88 optical camera acquires a number of position points, including, for example: P$_{trans1}$, i.e., a first end of the transducer array 68; P$_{trans2}$, i.e., a second end of the transducer array 68; and P$_{plane}$, i.e., a point on the transducer array 68 that is not collinear with P$_{trans1}$ and P$_{trans2}$ (Block 104). The homogeneous transformation between OP and W, $T_{OP}^{W}$, is then recorded (Block 106). The plurality of calibration parameters are then calculated (Block 108) from the measured number of points and the transformation, $T_{OP}^{W}$, as follows:

$$\mathbf{T_W^{OP}} = (\mathbf{T_{OP}^W})^{-1} \tag{1}$$

$$\mathbf{P_{trans-origin}} = \mathbf{T_W^{OP}}\mathbf{P_{trans1}} \tag{2}$$

$$\mathbf{L_{trans}} = \|\mathbf{P_{trans2}} - \mathbf{P_{trans1}}\| \tag{3}$$

$$\mathbf{\hat{u}_x} = \mathbf{T_W^{OP}} \frac{\mathbf{P_{trans2}} - \mathbf{P_{trans1}}}{\|\mathbf{P_{trans2}} - \mathbf{P_{trans1}}\|} \tag{4}$$

$$\hat{u}_y = \frac{(P_{plane} - P_{trans1}) \times (P_{trans2} - P_{trans1})}{\|(P_{plane} - P_{trans1}) \times (P_{trans2} - P_{trans1})\|} \tag{5}$$

With the plurality of calibration parameters determined, the hybrid probe 94 may be used to scan a portion of a patient's musculoskeletal system while the position sensor 88 tracks the physical movement of the hybrid probe 94.

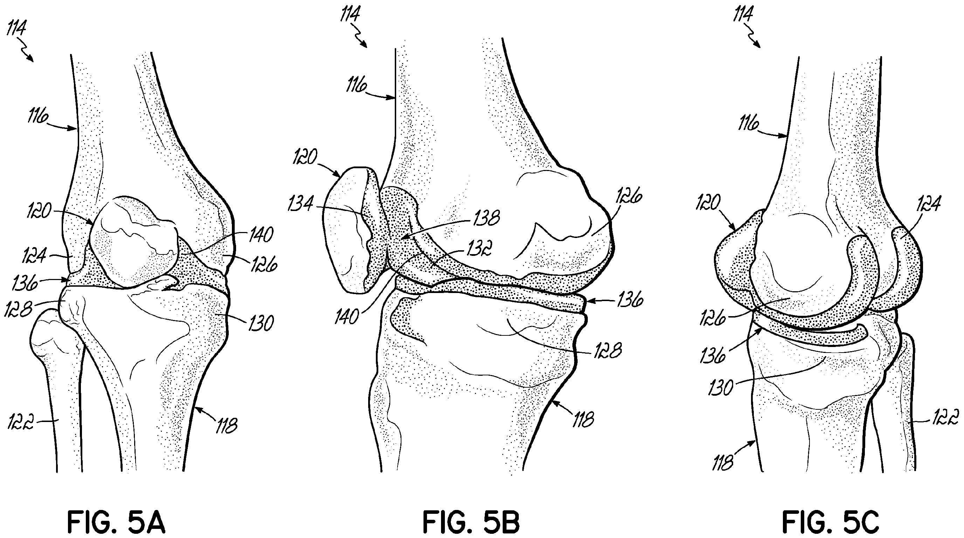

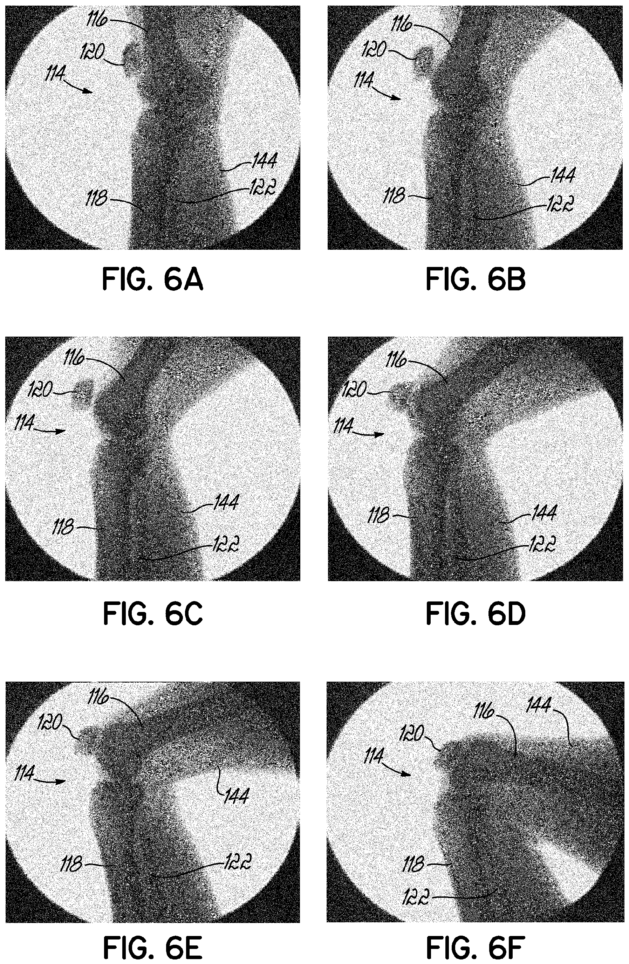

Because of the high reflectivity and attenuation of bone to ultrasound, ultrasound energy typically does not penetrate bone tissues to any significant degree. Therefore, soft tissues lying behind bone cannot be imaged and poses a challenge to ultrasound imaging of a joint. For example, as shown in FIGS. 5A-5C, the knee joint 114 is formed of three articulating bones: the femur 116, the tibia 118, and the patella 120, with the fibula 122 shown as environment. These bones 116, 118, 120 articulate together in two sub-joints: (1) the tibio-femoral joint 136 is formed by the articulation of the femur 116 with the tibia 118 at the respective condyles 124, 126, 128, 130 and (2) the patello-femoral joint 138 is formed by the articulation of the patella 120 with the femur 116 at the patellar surface 132 of the femur 116 and the articular surface 134 of the patella 120. During flexion-extension motions of the knee joint 114, portions of one or more articulating surfaces of the bones 116, 118, 120 are visible to the ultrasound beam, while other articulating surfaces are occluded. FIGS. 6A-6F include various fluoroscopic images of one patient's knee joint 114, showing the articulating surfaces at a plurality of degrees of flexion.

To acquire ultrasound images of a majority of the articulating surfaces, at least two degrees of flexion are required, including, for example, a full extension (FIG. 6A) and a deep knee bend (FIG. 6F) (or 90° flexion (FIG. 6E) if a deep knee bend is too difficult for the patient to achieve). That is, when the knee joint 114 is in the full extension (FIG. 6A), the posterior portions of the distal femur 116 and the proximal tibia 118 are accessible to the ultrasound beam. When the knee joint 114 is in the deep knee bend (FIG. 6F), the anterior surface of the distal femur 116, the trochlear grove 140, most of the inferior surface of the femoral condyles 124, 126, the anterior superior surface of the tibia 118, and the anterior surface of the tibia 118 are accessible to the ultrasound beam. Both the medial and lateral parts of the femur 116 and tibia 118 are visible at all flexion angles of the knee joint 114.

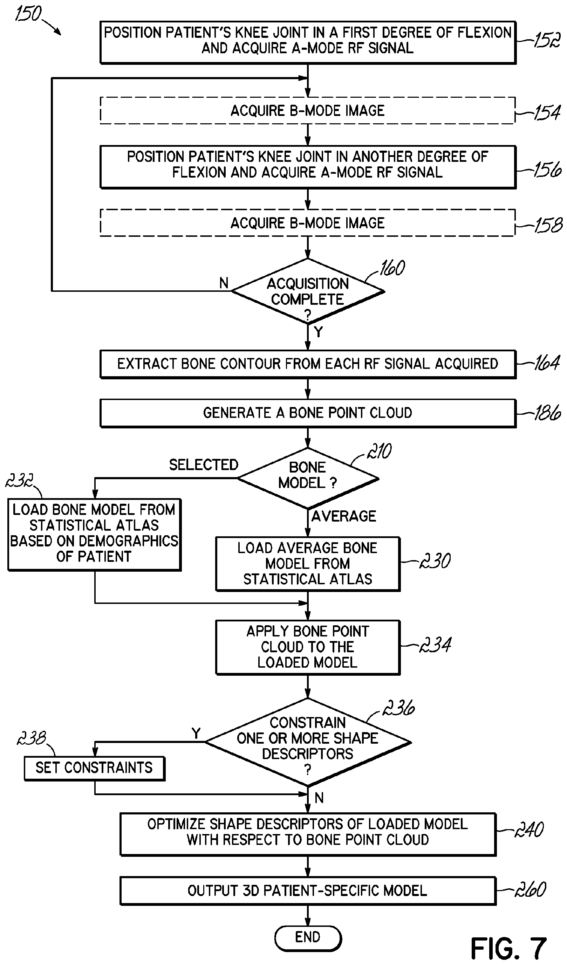

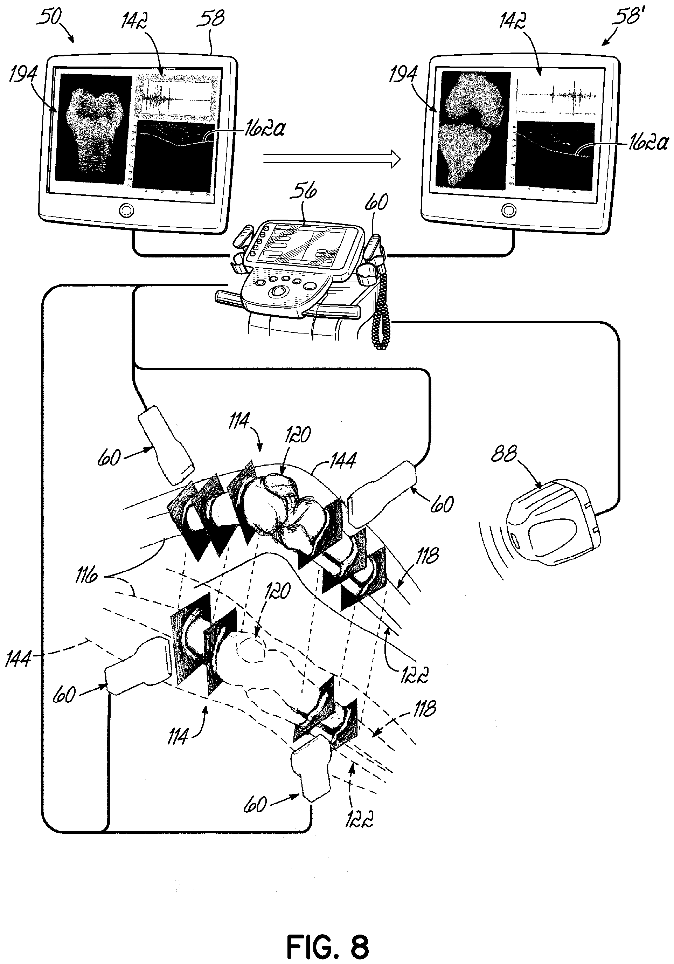

Turning now to FIG. 7, one method 150 of acquiring data for construction of a 3-D patient-specific bone model in accordance with aspects of the invention is described. The method begins with acquiring a plurality of RF signals from an A-mode ultrasound beam scan of a bone. To acquire the RF signals for creating the 3-D patient-specific model of the knee joint 114, the patient's knee joint 114 is positioned and held in one of the two or more degrees of flexion (Block 152). The hybrid probe 94 is positioned, at two or more locations, on the patient's epidermis 144 adjacent to the knee joint 114 for acquisition of the A-mode RF signal 142, one example, as shown in FIG. 8. Although the acquired signal includes a plurality of RF signals, for convenience, the RF signals are sometimes referred to herein in singular form.

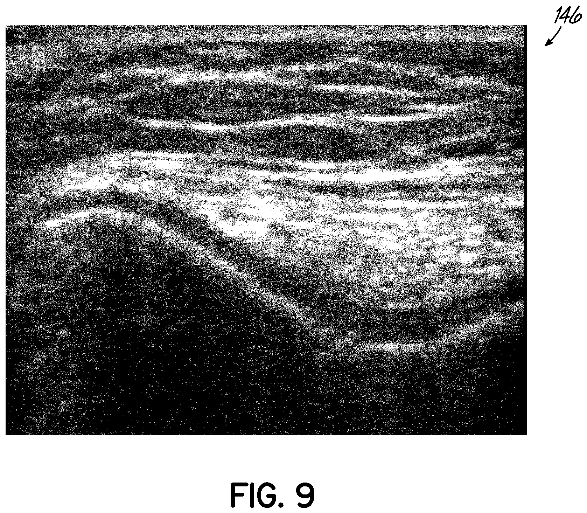

As shown in FIG. 8, with continued reference to FIG. 7, the position of the patient's knee joint 114 is held stationary to avoid motion artifacts during image acquisition. Should motion occur, scans may be automatically aligned to the statistically-most likely position given the data acquired. Furthermore, holding the knee stationary and compensating for movement removes the need for invasive fiducial bone markers or high-error skin markers. In some embodiments, B-mode images, similar to the one shown in FIG. 9, may also be processed from the gathered data (Block 154) for subsequent visualization and overlain with the bone contours, as described in detail below.

When the RF signal 142, and if desired B-mode image, acquisition is complete for the first degree of flexion, the patient's knee 114 is moved to another degree of flexion and the reflected RF signal 142 acquired (Block 156). Again, if desired, the B-mode image may also be acquired (Block 158). The user then determines whether acquisition is complete or whether additional data is required (Block 160). That is, if visualization of a desired surface of one or more bones 116, 118, 120 is occluded ("NO" branch of decision block 160), then the method returns to acquire additional data at another degree of flexion (Block 156). If the desired bone surfaces are sufficiently visible ("YES" branch of decision block 160), then the method 150 continues.

FIG. 8 illustrates acquisition of the RF signal 142 in yet another manner. That is, while the patient's leg is in full extension (shown in phantom), the hybrid probe 94 is positioned at two or more locations on the patient's epidermis 144 adjacent to the knee joint 114. The patient's leg is then moved to a second degree of flexion (90° flexion is shown in solid) and the hybrid probe 94 again positioned at two or more locations on the patient's epidermis 144. All the while, the position sensor 88 tracks the location of the hybrid probe 94 in the 3-D space. Resultant RF signal profiles, bone models, bone contours, and so forth may be displayed on the monitor 58 during and the monitor 58' after the model reconstruction.

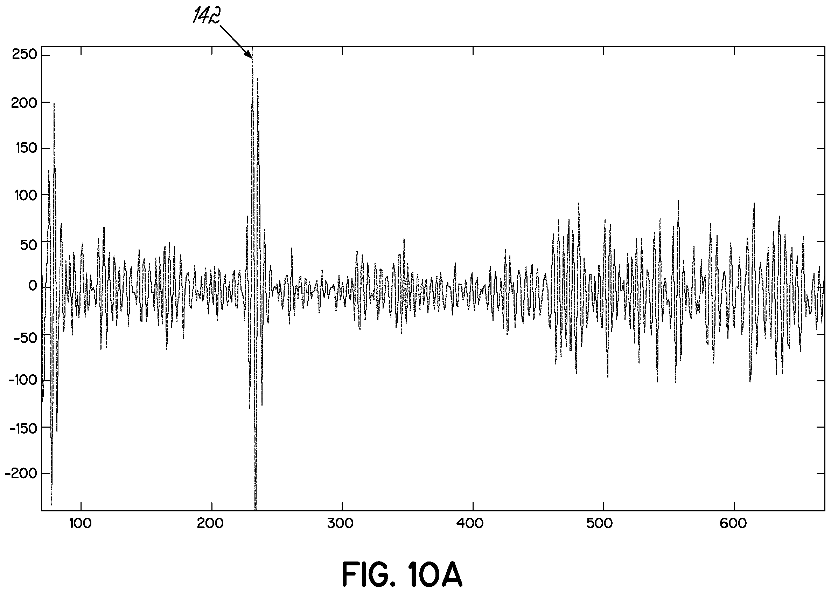

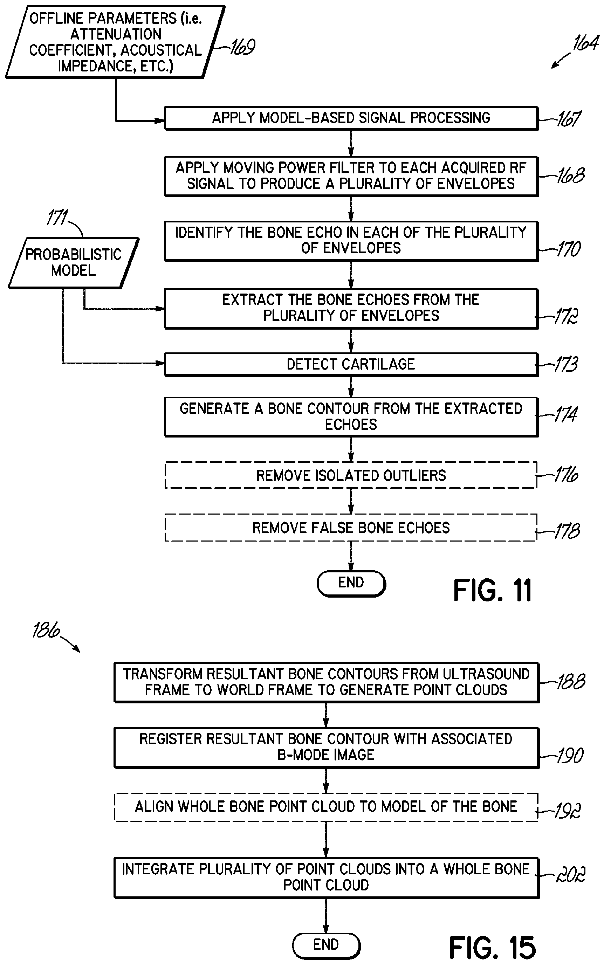

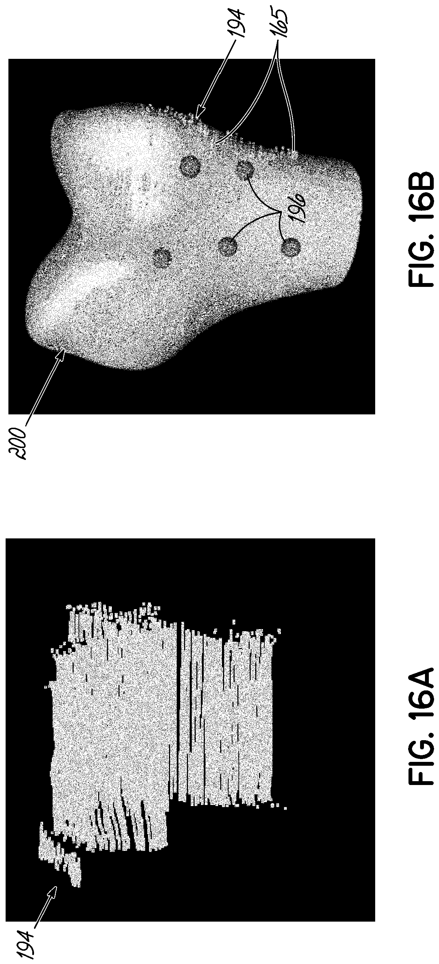

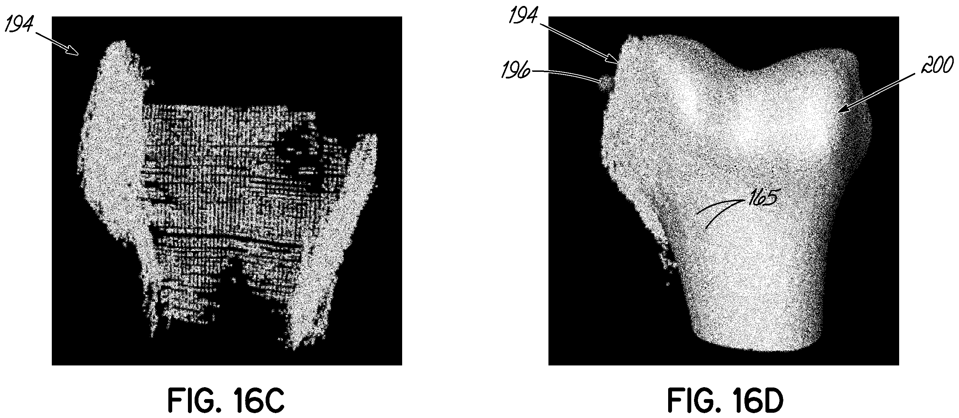

After all data and RF signal acquisition is complete, the computer 54 is operated to automatically isolate that portion of the RF signal, i.e., the bone contour, from each of the plurality of RF signals. In that regard, the computer 54 may sample the echoes comprising the RF signals to extract a bone contour for generating a 3-D point cloud 165 (FIG. 16B) (Block 164). More specifically, and with reference now to FIGS. 10A-10E and 11, and with continued reference to FIGS. 7-9, one method of extracting the bone contours from each of the RF signal 142 is shown. FIG. 10A illustrates one exemplary, raw RF signal 142 as acquired by one transducer comprising the transducer array 68 of the ultrasound probe portion of the hybrid probe 94. Each acquired raw, RF signal includes a number of echoes 162, wherein the echoes 162 may be isolated, partially overlapping, or fully overlapping. Each of the plurality of echoes originates from a reflection of at least a portion of the ultrasound energy at an interface between two tissues having different reflection and/or attenuation coefficients, as described in greater detail below.

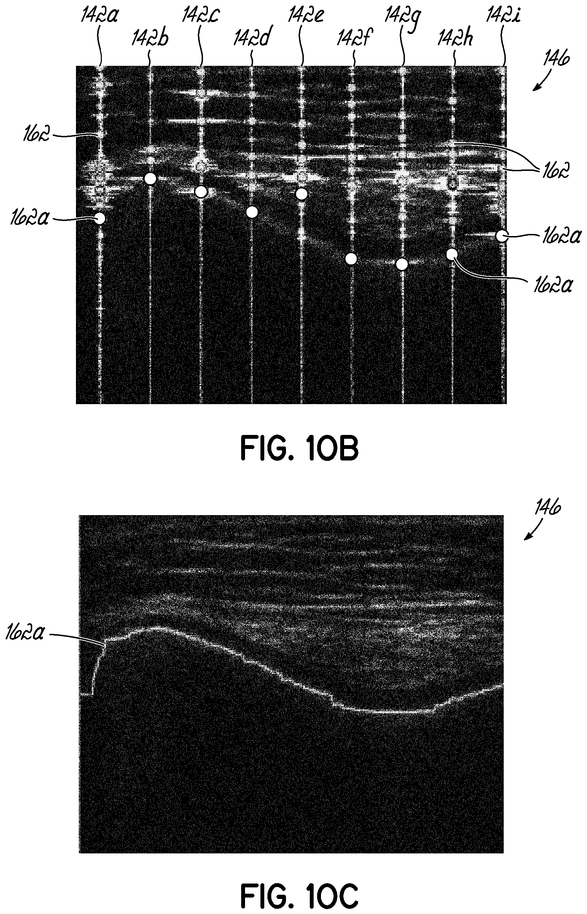

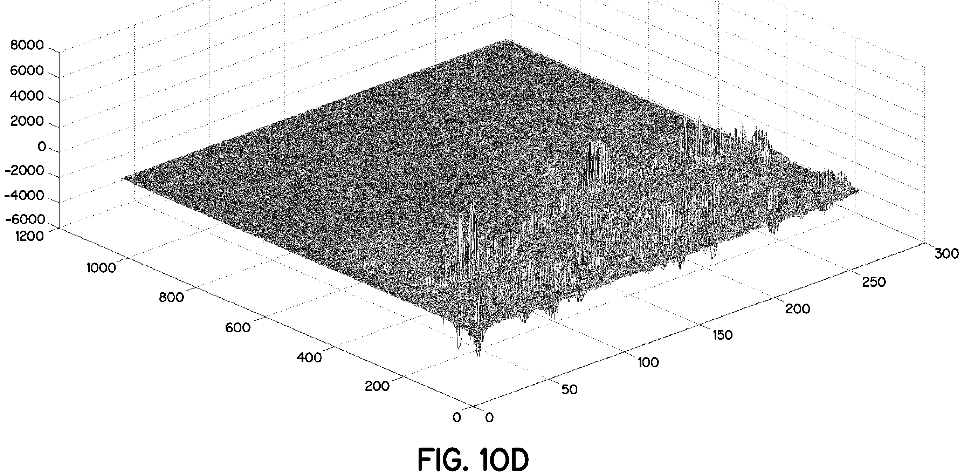

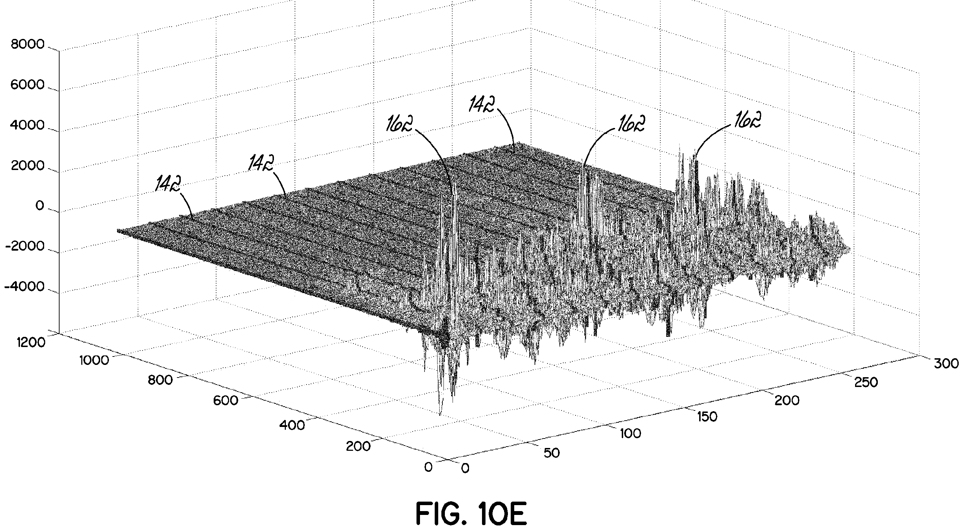

FIGS. 10B and 10C illustrate an ultrasound frame 146 having select ones of the raw RF signals 142 with some echoes 162 identified. FIGS. 10D and 10E are 3-D renderings of 2D images taken from an ultrasound frame 146 with select ones of the RF signals 142 identified in FIG. 10E.

Referring specifically now to FIG. 11, the method of extracting the bone contour 162a begins with a model-based signal processing approach incorporating a *priori* knowledge of an underlying physical problem into a signal processing scheme. In this way, the computer 54 may process the RF signal 142 and remove some preliminary noise based on an estimated, or anticipated, result. For example, with ultrasound signal acquisition, the physical problem is represented by the governing waveform equation, such as described in VARSLOT T, et al., "Computer Simulation of Forward Wave Propagation in Soft Tissue," IEEE Transactions on Ultrasonics, Ferroelectrics, and Frequency Control, 1473-1482:52(9), Sept. 2005. The wave equation describes the propagation behavior of the ultrasonic wave in a heterogeneous medium. The solution to the wave equation may be represented as a state-space model-based processing scheme, such as described in CHEN Z, et al., "Bayesian Filtering: From Kalman Filters to Particle Filters, and Beyond," Statistics, 1-69, retrieved from http://citeseerx.ist.psu.edu/viewdoc/download?doi=10.1.1.107.7415&rep=rep1&type =pdf, accessed Aug. 2011 . In accordance with one embodiment of the present invention, a general solution to the model-based ultrasound wave estimator problem is developed using Bayesian estimators (e.g., maximum *a posteriori*), which leads to a nonlinear model-based design.

The model-based signal processing of the RF signal 142 begins with enhancing the RF signal by applying the model-based signal processing (here, the Bayesian estimator) (Block 167). To apply the Bayesian estimator, offline measurements are first collected from phantoms, cadavers, and/or simulated tissues to estimate certain unknown parameters, for example, an attenuation coefficient (i.e., absorption and scattering) and an acoustic impedance (i.e., density, porosity, compressibility), in a manner generally described in VARSLOT T (refer above). The offline measurements (Block 169) are input into the Bayesian estimator and the unknown parameters are estimated as follows:

$$z = h(x) + v \qquad (6)$$

$$P(t) = e^{(-\beta t^2)} \cdot \cos\left(2\pi \cdot f_0 \cdot t\right) \qquad (7)$$

Where *h* is the measurement function that models the system and *v* is the noise and modeling error. In modeling the system, the parameter, *x*, that best fits the measurement, *z*, is determined. For example, the data fitting process may find an estimate of $\hat{x}$ that best fits the measurement of *z* by minimizing some error norm, $\|\varepsilon\|$, of the residual, where:

$$\varepsilon = z - h(\hat{x}) \qquad\qquad (8)$$

For ultrasound modeling, the input signal, *z*, is the raw RF signal from the offline measurements, the estimate $h(\hat{x})$ is based on the state space model with known parameters of the offline measurements (i.e., density, etc.). The error, *v*, may encompass noise, unknown parameters, and modeling errors in an effort to reduce the effect of *v* by minimizing the residuals and identifying the unknown parameters form repeated measurements. Weighting the last echo within a scan line by approximately 99%, as bone, is one example of using likelihood in a Bayesian framework. A Kalman filter may alternatively be used, which is a special case of the recursive Bayesian estimation, in which the signal is assumed to be linear and have a Gaussian distribution.

It would be readily appreciated that the illustrative use of the Bayesian model here is not limiting. Rather, other model-based processing algorithms or probabilistic signal processing methods may be used within the spirit of the present invention.

With the model-based signal processing complete, the RF signal 142 is then transformed into a plurality of envelopes to extract the individual echoes 162 existing in the RF signal 142. Each envelope is determined by applying a moving power filter to each RF signal 142 (Block 168) or other suitable envelope detection algorithm. The moving power filter may be comprised of a moving kernel of a length that is equal to the average length of an individual ultrasound echo 162. With each iteration of the moving kernel, the power of the RF signal 142 at the instant kernel position is calculated. One exemplary kernel length may be 20 samples; however, other lengths may also be used. The value of the RF signal 142 represents the value of the signal envelope at that position of the RF signal 142. Given a discrete-time signal, *X*, having a length, *N*, each envelope, *Y*, using a moving power filter having length, *L*, is defined by:

$$Y_k = \sum_{i=k-\frac{L}{2}}^{k+\frac{L}{2}} X_i^2 \; \forall \, k \, \epsilon \left[ \frac{L}{2}, N - \frac{L}{2} - 1 \right] \qquad\qquad (9)$$

In some embodiments, this and subsequent equations use a one-sided filter of varying length for the special cases of the samples before the $\frac{L}{2}$ sample (left-sided filter), and after the $N - \frac{L}{2} - 1$ sample (right-sided filter).

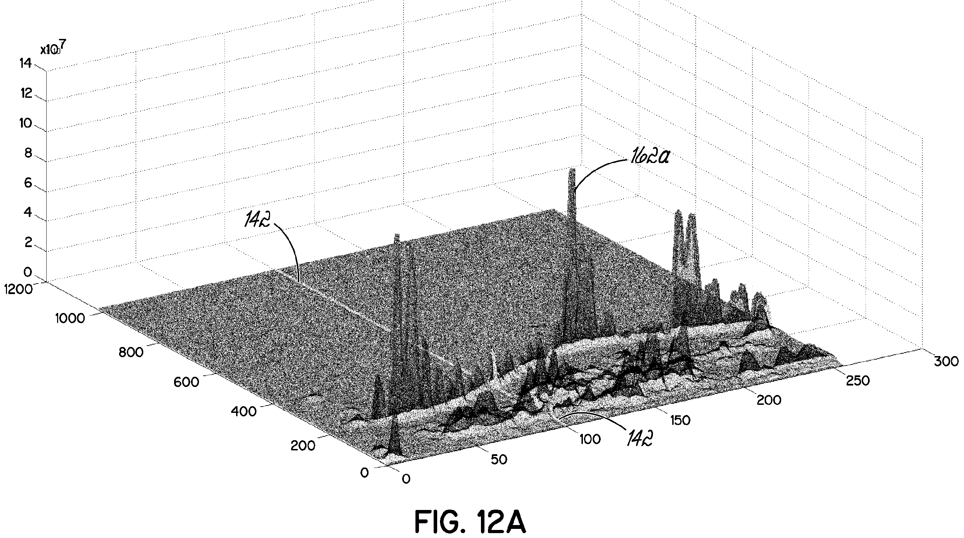

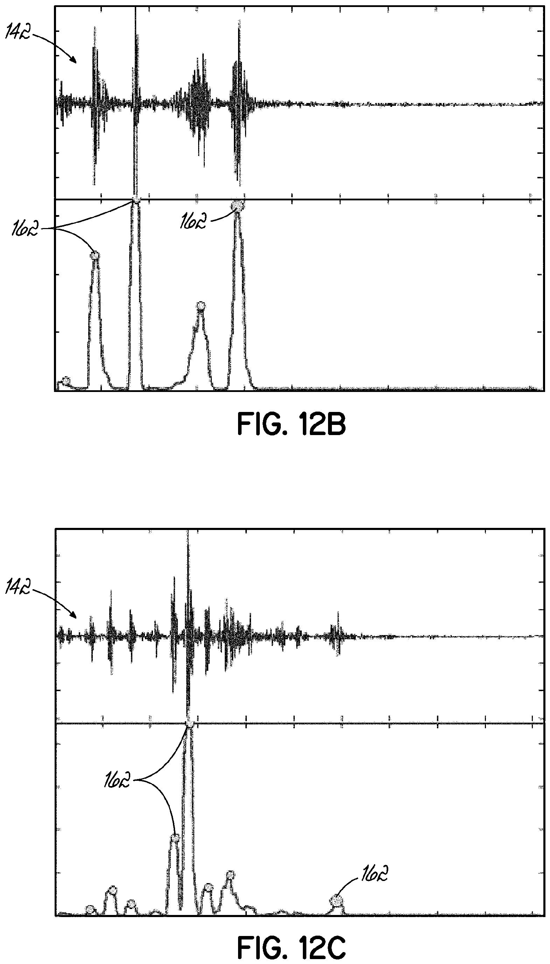

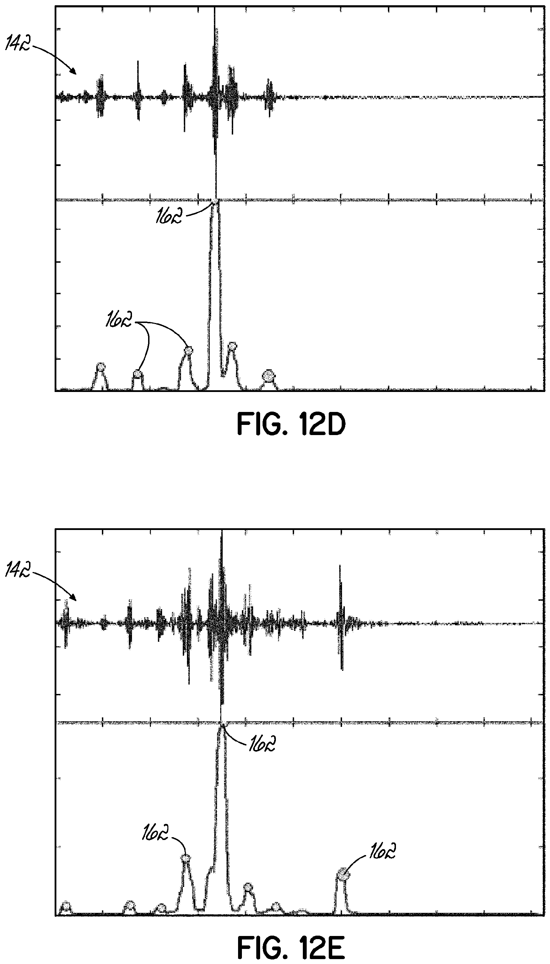

Each envelope produced by the moving power filter, shown in FIG. 10B, includes a plurality of local peaks (identified in FIG. 10B as enlarged dots at the intersection of each envelope with an echo 162), each being a clear representation of the individual echoes 162 existing in the acquired RF signal 142 for the various tissue interfaces. As an example of such process, FIGS. 12A-12D more clearly illustrate the RF signal 142 (top in each figure) at four iterations of the kernel of the moving power filter as well as the corresponding envelope (bottom in each figure). Individual echoes 162 in each envelope are again identified with an enlarged dot.

Of the plurality of echoes 162 in the RF signal 142, one echo 162 is of particular interest, e.g., the echo corresponding to the bone-soft tissue interface. This bone echo (hereafter referenced as 162a) is generated by the reflection of the ultrasound energy at the surface of the scanned bone. More particularly, the soft tissue-bone interface is characterized by a high reflection coefficient of 43%, which means that 43% of the ultrasound energy reaching the surface of the bone is reflected back to the transducer array 68 of the ultrasound probe 60 (FIG. 2). This high reflectivity gives bone the characteristic hyper-echoic appearance in an ultrasound image.

Bone is also characterized by a high attenuation coefficient of the applied RF signal (6.9 db/cm/mHz for trabecular bone and 9.94 db/cm/mHz for cortical bone). At high frequencies, such as those used in musculoskeletal imaging (that is, in the range of 7-14 MHz), the attenuation of bone becomes very high and the ultrasound energy ends at the surface of the bone. Therefore, an echo 162a corresponding to the soft-tissue-bone interface is the last echo 162a in the RF signal 142. The bone echo 162a is identified by selecting the last echo having a normalized envelope amplitude (with respect to a maximum value existing in the envelope) above a preset threshold (Block 170).

The bone echoes 162a are then extracted from each frame 146 (Block 172) and used to generate the bone contour existing in that RF signal 142 and as shown in FIG. 10C (Block 174). In extracting the bone echoes, a probabilistic model (Block 171) may be input and applied to the RF signals 142 of each frame 146. The probabilistic model (Block 171) may further be used in detecting cartilage within the envelopes of the RF signals 142 (Block 173). While the probabilistic signal processing method may include the Bayesian estimator described previously, in still other embodiments, the signal processing may be, a maximum likelihood ratio, neural network, or a support vector machine ("SVM"), for example, with the latter of which is further described below.

Prior to implementing the SVM, the SVM may be trained to detect cartilage in RF signals. One such way of training the SVM includes information acquired from a database comprising of MRI images and/or RF ultrasound images to train the SVM to distinguish between echoes associated with cartilage from the RF signals 142, and from within the noise or in ambiguous soft tissue echoes. In constructing the database in accordance with one embodiment, knee joints from multiple patient's are imaged using both MRI and ultrasound. A volumetric MRI image of each knee joint is reconstructed, processed, and the cartilage and the bone tissues are identified and segmented. The segmented volumetric MRI image is then registered with a corresponding segmented ultrasound image (wherein bone tissue is identified). The registration provides a transformation matrix that may then be used to register the raw RF signals 142 with a reconstructed MRI surface model.

After the raw RF signals 142 are registered with the reconstructed MRI surface model, spatial information from the volumetric MRI images with respect to the cartilage tissue may be used to determine the location of a cartilage interface on the raw RF signal 142 over the articulating surfaces of the knee joint.

The database of all knee joint image pairs (MRI and ultrasound) is then used to train the SVM. Generally, the training includes loading all raw RF signals, as well as the location of the bone-cartilage interface of each respective RF signal. The SVM may then determine the location of the cartilage interface in an unknown, input raw RF signal. If desired, a user may chose from one or more kernels to maximize a classification rate of the SVM.

In use, the trained SVM receives a reconstructed knee joint image of a new patient as well as the raw RF signals. The SVM returns the cartilage location on the RF signal data, which may be used, along with the tracking information from the tracking system (e.g., the optical tracking marker 86 and the position sensor 88) to generate 3-D coordinates for each point on the cartilage interface. The 3-D coordinates may be triangulated and interpolated to form a complete cartilage surface.

Referring still to FIG. 11, the resultant bone contours may be noisy and require filtering to remove echoes 162 that may be falsely detected as the bone echo 162a. Falsely detected echoes 162 may originate from one of at least two sources: (1) an isolated outlier echoes and (2) a false bone echoes. Furthermore, some images may not include a bone echo 162a; therefore any detected echo 162 is noise and should be filtered out. Therefore, proper determination of the preset threshold or filtering algorithm may prevent the false selection of a falsely detected echo 162.

Isolated outliers are those echoes 162 in the RF signal 142 that correspond to a tissue interface that is not the soft-tissue-bone interface. Selection of the isolated outliers may occur when the criterion is set too high. If necessary, the isolated outliers may be removed (Block 176) by applying a median filter to the bone contour. That is, given a particular bone contour, $X$, having a length, $N$, with a median filter length, $L$, the median-filter contour, $Y_k$, is:

$$Y_k = Median \left[ X_{k-\frac{L}{2}}, X_{k+\frac{L}{2}} \right] \forall \ k \in \left[ \frac{L}{2}, N - \frac{L}{2} - 1 \right] \qquad (10)$$

False bone echoes are those echoes 162 resulting from noise or a scattering echo, which result in a detected bone contour in a position where no bone contour exists. The false bone echoes may occur when an area that does not contain a bone is scanned, the ultrasound probe 60 is not oriented substantially perpendicular with respect to the bone surface, the bone lies deeper than a selected scanning depth, the bone lies within the selected scanning depth but its echo is highly attenuated by the soft tissue overlying the bone, or a combination of the same. Selection of the false bone echoes may occur when the preset threshold is too low.

Frames 146 containing false bone echoes should be removed. One such method of removing the false bone echoes (Block 178) may include applying a continuity criteria. That is, because the surface of the bone has a regular shape, the bone contour, in the two-dimensions of the ultrasound image, should be continuous and smooth. A false bone echo will create a non-continuity, and exhibits a high degree of irregularity with respect to the bone contour.

One manner of filtering out false bone echoes is to apply a moving standard deviation filter; however, other filtering methods may also be used. For example, given the bone contour, $X$, having a length, $N$, with a median filter length, $L$, the standard deviation filter contour:

$$Y_k = \sqrt{\frac{1}{L-1}\sum_{i=k-\frac{L}{2}}^{i=k-\frac{L}{2}}(X_i - \bar{X})^2} \quad \forall \ k \in \left[\frac{L}{2}, N - \frac{L}{2} - 1\right] \qquad (11)$$

Where $Y_k$ is the local standard deviation of the bone contour, which is a measure of the regularity and continuity of the bone contour. Segments of the bone contour including a false bone echo are characterized by a higher degree of irregularity and have a high $Y_k$ value. On the other hand, segments of the bone contour including only echoes resulting from the surface of the bone are characterized by high degree regularity and have a low $Y_k$ value.

A resultant bone contour 180, resulting from applying the moving median filter and the moving standard deviation filter, includes a full length contour of the entire surface of the bone, one or more partial contours of the entire surface, or contains no bone contour segments.

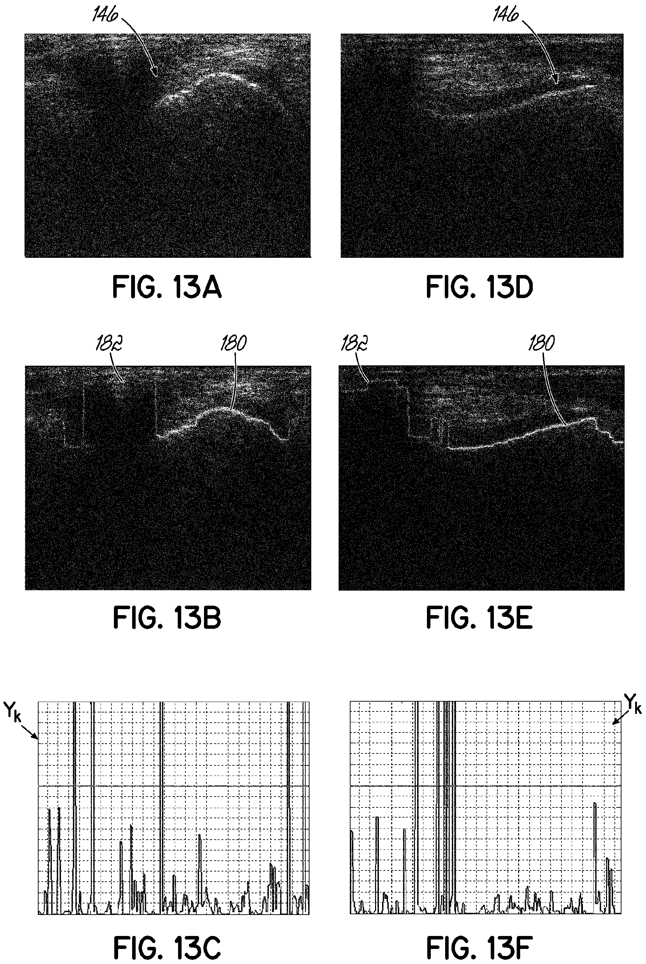

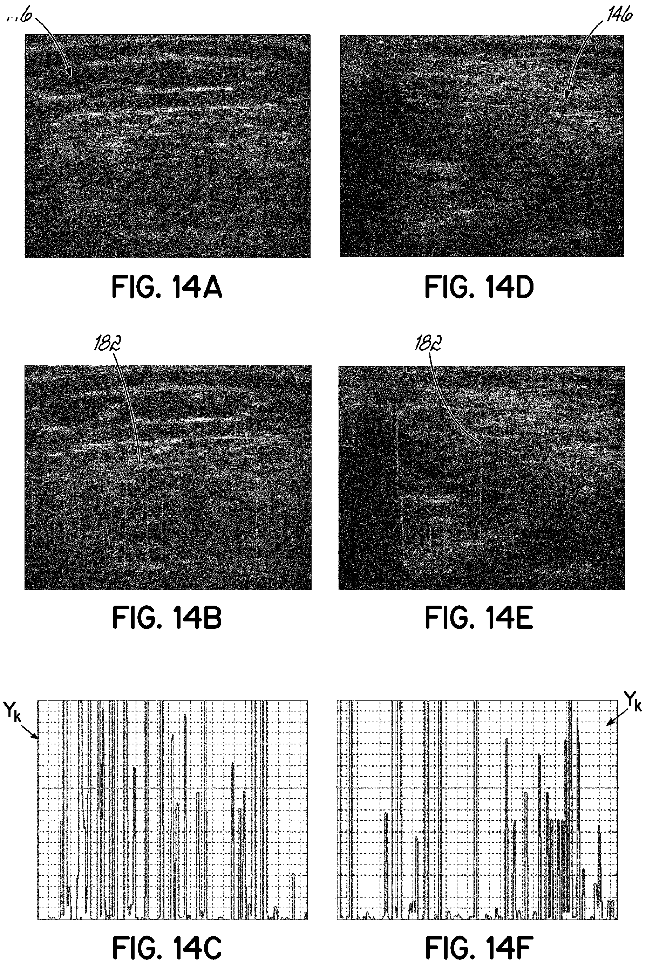

FIGS. 12A-12F and 13A-13F illustrate the resultant bone contour 180 that is selected from those segments of the extracted bone contour that satisfy two conditions: (1) the continuity criteria, having a local standard deviation value below selected standard deviation threshold, and (2) a minimum-length criteria, which avoids piecewise-smooth noise contour segments from being falsely detected as bone contour. In some exemplary embodiments, the length of the standard deviation filter may be set to 3 and the threshold set to 1.16 mm, which may correspond to 30 signal samples. Accordingly, FIGS. 13A and 13D illustrate two exemplary RF signals 142 with the resultant bone contours 180 extracted and filtered from the noise 182 (including isolated outliers and false body echoes), shown in FIGS. 13B and 13E, respectively. FIGS. 13C and 13F respectively illustrate the standard deviation, $Y_k$, calculated as provided in Equation 11 above. FIGS. 14A-14F are similar to FIGS. 13A-13F, but include two exemplary RF signals 142 in which no bone tissue was scanned.

With the bone contours isolated from each of the RF signals, the bone contours may now be transformed into a point cloud. For instance, returning now to FIG. 7, the resultant bone contours 180 may then undergo registration with the optical system to construct a bone point cloud 194 representing the surface of at least a portion of each scanned bone (Block 186), which is described herein as a multiple step registration process. In one embodiment, the process is a two-step registration process. The registration step (Block 186) begins by transforming the resultant bone contour 180 from a 2D contour in the ultrasound frame into a 3-D contour in the world frame (Block 188). This transformation is applied to all resultant bone contours 180 extracted from all of the acquired RF signals 142.

To transform the resultant bone contour 180 into the 3-D contour, each detected bone echo 162a undergoes transformation into a 3-D point as follows:

$$d_{echo} = n_{echo} T_s C_{us} \qquad (12)$$

$$l_{echo} = L_{trans}\frac{n_{line}}{N_{lines}}\hat{u}_x \qquad (13)$$

$$P_{echo}^{OP} = P_{trans-origin} + d_{echo}\hat{u}_y + l_{echo}\hat{u}_x \qquad (14)$$

$$P_{echo}^{W} = H_{OP}^{W}\, P_{echo}^{OP} \qquad (15)$$

Where the variables are defined as follows:

| | |
|---|---|
| $d_{echo}$ | depth of the bone echo (cm) |
| $n_{echo}$ | sample index of the detected bone echo |
| $T_S$ | RF signal sampling period (sec/sample) |
| $C_{us}$ | speed of ultrasound in soft tissue (154 x $10^3$ cm/s) |
| $l_{echo}$ | distance from the $P_{trans-origin}$ (FIG. 2) of the transducer array 68 (FIG. 2) to the current scan line (cm) |

(continued)

| | |
|---|---|
| $P_{echo}^{OP}$ | detected point on the bone surface represented in the local frame |
| $n_{line}$ | index of the scan line containing the bone echo in the image |
| | |
| $N_{lines}$ | number of scan lines in the image |
| $P_{echo}^{W}$ | detected surface of the bone relative to the world frame |
| $H_{OP}^{W}$ | homogeneous transformation between the local frame and the world frame, as described previously |
| $H_{OP}^{W}$ | dynamically obtained transformation that contains the position and orientation of the optical tracking marker 86 (FIG. 2) |

If so desired, an intermediate registration process may be performed between the resultant bone contour and a B-mode image, if acquired (Block 190). This registration step is performed for visualizing the resultant bone contour 180 with the B-mode image (FIG. 9), which provides visual validation and feedback of the resultant bone contour 180 detection process, in real time, while the user is performing the scan. This visual validation may aid the user in determining whether acquisition is completed (Block 160), as described previously. More specifically, the resultant bone contour 180 is registered with the B-mode image by:

$$P_{echo}^{I} = (l_{echo}I_x \quad d_{echo}I_y) \qquad (16)$$

Where $I_x$ and $I_y$ denote the B-mode image resolution (pixels/cm) for the x- and y-axes respectively. $P_{echo}^{I}$ denotes the coordinates of the bone contour point relative to the ultrasound frame.

After the resultant bone contours 180 are transformed and, if desired, registered (Block 190) (FIG. 15), the plurality of point clouds 165 (FIG. 16B) are generated representing the surface of the bone. During the second registration process the plurality of point clouds 165 are integrated into a bone point cloud 194 representing the entire surface of the scanned bone.

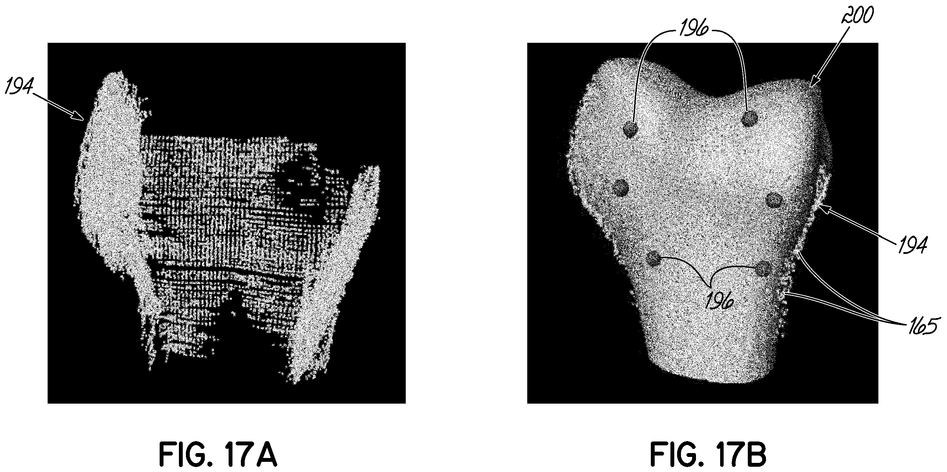

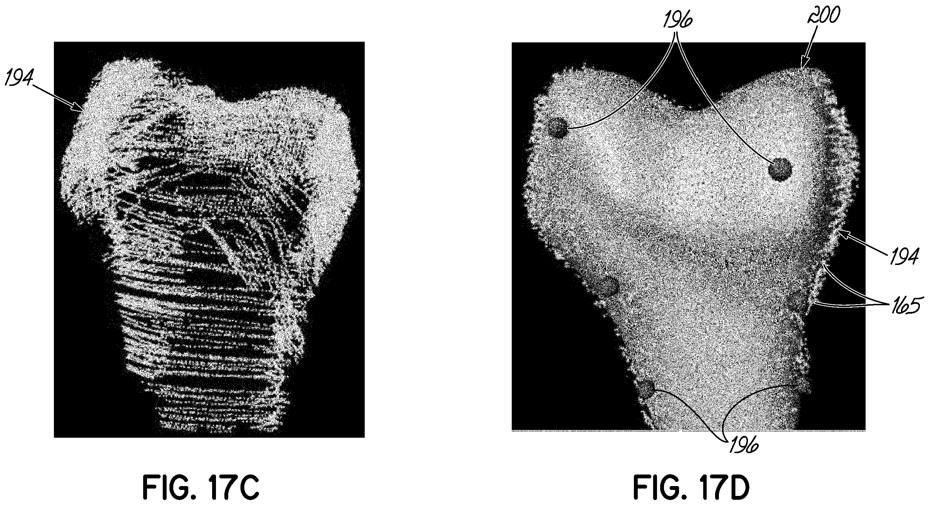

To begin the second registration process, as shown in FIGS. 16A-17D, the plurality of point clouds 194 are initially aligned to a standardized model of the scanned bone, here a model femur 200, for example, by using 4-6 previously specified landmarks 196 (Block 192). More specifically, the user may identify the plurality of landmarks 196 on the model femur 200, which need not be identified with high accuracy. After this initial alignment, an iterative closest point ("ICP") alignment is performed to more accurately align the plurality of point clouds to the standardized model. If necessary, noise may be removed by thresholding for a distance between a respective point of the plurality of point clouds and the closest vertices in the model femur 200; however, other filtering methods may alternatively be used. For instance, an average distance plus one standard deviation may be used as the threshold. The process is repeated for each point cloud 165 of the plurality for the surface of the scanned bone. The now aligned point clouds 165 are then integrated into a single uniform point cloud 194 that represents the surface of the scanned bone (Block 202).

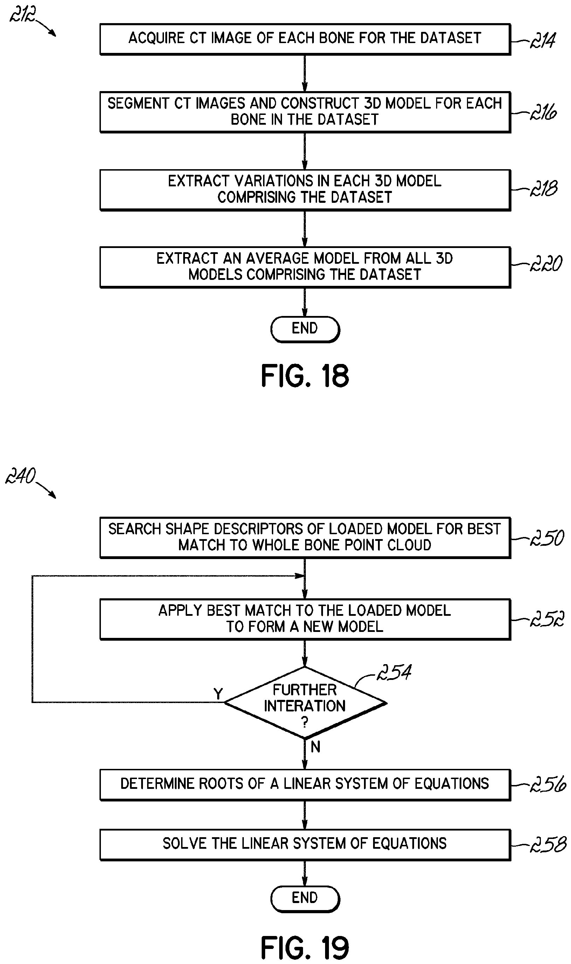

After the point clouds 194 are formed, a bone model may be optimized in accordance with the point clouds 194. That is, the bone point cloud 194 is then used to reconstruct a 3-D patient-specific model of the surface of the scanned bone. The reconstruction begins with a determination of a bone model from which the 3-D patient-specific model is derived (Block 210). The bone model may be a generalized model based on multiple patient bone models and may be selected from a principle component analysis ("PCA") based statistical bone atlas. One such *a priori* bone atlas, formed in accordance with the method 212 of FIG. 18, includes a dataset of 400 dry femur and tibia bone pairs, scanned by CT (Block 214) and segmented to create models of each bone (Block 216). The method of building and using one such statistical atlas is described in MAHFOUZ M et al., "Automatic Methods for Characterization of Sexual Dimorphism of Adult Femora: Distal Femur," Computer Methods in Biomechanics and Biomedical Engineering, 10(6) 2007. Each bone model, $M_i$, (where $I \in [1, N]$, N being the number of models in the dataset) has the same number of vertices, wherein the vertex, $V_j$, in a select one model corresponds (at the same anatomical location on the bone) to the vertex, $V_j$, in another one model within the statistical atlas.

PCA is then performed on each model in the dataset to extract the modes of variation of the surface of the bone (Block 218). Each mode of variation is represented by a plurality of eigenvectors resulting from the PCA. The eigenvectors, sometimes called eigenbones, define a vector space of bone morphology variations extracted from the dataset. The PCA may include any one model from the dataset, expressed as a linear combination of the eigenbones. An average model of all of the 3-D models comprising the dataset is extracted (Block 220) and may be defined as:

$$M_{avg} = \frac{1}{N} \sum_{i=1}^{N} M_i \qquad (17)$$

$$M_i = M_{avg} + \sum_{k=1}^{L} \alpha_{ik} U_k \quad \forall i \epsilon \, [1, N] \qquad (18)$$

Where the variables are defined as follows:

| | |
|---|---|
| $M_{avg}$ | is the mean bone of the dataset |
| $L$ | dimensionality of the eigenspace (i.e., the number of eigenbones) and is equal to N |
| $N$ | number of models in the data |
| $U_k$ | $k^{th}$ eigenbone |
| $\alpha_{ik}$ | $k^{th}$ shape descriptor or eigenbone's coefficient for the $i^{th}$ model |

Furthermore, any new model, $M_{new}$ (i.e., a model not already existing in the dataset), may be approximately represented by new values of the shape descriptors (eigenvectors coefficients) as follows:

$$M_{new} \simeq M_{avg} + \sum_{k=1}^{W} \alpha_k U_k \qquad (19)$$

Where the variables are defined as follows:

| | |
|---|---|
| $M_{new}$ | new bone model |
| $\alpha_k$ | indexed shape descriptors for the new model |
| $W$ | number of principal components to use in the model approximation, where $W \leq L$ |

The accuracy of $M_{new}$ is directly proportional to the number of principal components ($W$) used in approximating the new model and the number of models, $L$, of the dataset used for the PCA. The residual error or root mean square error ("RMS") for using the PCA shape descriptors is defined by:

$$RMS = rms \left[ M_{new} - (M_{avg} + \sum_{k=1}^{W} \alpha_k U_k) \right] \qquad (20)$$

Therefore, the RMS when comparing any two different models, A and B, having the same number of vertices is defined by:

$$RMS = rms(A - B) = \sqrt{\frac{\sum_{j=1}^{m} \|V_{Aj} - V_{Bj}\|^2}{m}} \qquad (21)$$

Where $V_{Aj}$ is the $j^{th}$ vertex in model A, and similarly, $V_{Bj}$ is the $j^{th}$ vertex in model B.

Returning again to FIG. 7, the average model ("AVERAGE" branch of Block 210) is loaded (Block 230) or a subset model is selected ("SELECTED" branch of Block 210) from the statistical atlas based on demographics that are similar to the patient and loaded (Block 232) for optimization. The bone point cloud 194 is then applied to the loaded model (Block 234) so that the shape descriptors of the loaded model may be changed to create the 3-D patient-specific model. If desired, one or more shape descriptors may be constrained ("YES" branch of Block 254) so that the 3-D patient-specific model will have the same anatomical characteristics as the loaded model. Accordingly, the one or more shape descriptors are set (Block 238). With the constraints set, the loaded model may be deformed (or optimized) (Block 240) into a model that resembles the appropriate bone and not an irregularly, randomly shaped model. If no constraints are desired ("NO" branch of Block 240) and then the loaded model is optimized (Block 240).

Changing the shape descriptors to optimize the loaded model (Block 240) may be carried out by one or more optimization algorithms, guided by a scoring function, to find the values of the principal components coefficients to create the 3-D patient-specific new model and are described with reference to FIG. 19. The illustrated optimization algorithm includes a two-step optimization method of successively-applied algorithms to obtain the 3-Dpatient-specific model that best fits the bone point cloud 194 as discussed below. Although a two-step method is described, the present invention is not limited to just a two-step optimization method.

The first algorithm may use a numerical method of searching the eigenspace for optimal shape descriptors. More specifically, the first algorithm may be an iterative method that searches the shape descriptors of the loaded model to find a point that best matches the bone point cloud 194 (Block 250). One such iterative method may include, for example, Powell's conjugate gradient descent method with a RMS as the scoring function. The changes are applied to the shape descriptors of the loaded model by the first algorithm to form a new model, $M_{new}$, (Block 252) defined by Equation 19. The new model, $M_{new}$, is then compared with the bone point cloud 194 and the residual error, $E$, calculated to determine whether a further iterative search is required (Block 254). More specifically, given a bone point cloud, Q, having $n$ points therein, and an average model, $M_{avg}$, with $l$ vertices, there may be a set of closest vertices, $V$, in the average model, $M_{avg}$ to the bone point cloud, $Q$.

$$v_i = \operatorname*{argmin}_{v_j \epsilon M} \lVert v_j - q_i \rVert \ \forall i \epsilon [1, n], j \epsilon [1, l] \qquad (22)$$

Where $v_i$ is the closest point in the set, $V$, to $q_i$ in the bone point cloud, $Q$. An octreemay be used to efficiently search for the closest points in $M_{new}$. The residual error, $E$, between the new model, $M_{new}$ and the bone point cloud, $Q$, is then defined as:

$$E = \lVert V - Q \rVert^2 \qquad (23)$$

With sufficiently high residual error ("YES" branch of Block 254), the method returns to further search the shape descriptors (Block 250). If the residual error is low ("NO" branch of Block 254), then the method proceeds.

The second algorithm of the two-step method refines the new model derived from the first algorithm by transforming the new model into a linear system of equations in the shape descriptors. The linear system is easily solved by linear system equation, implementing conventional solving techniques, which provide the 3-D patient-specific shape descriptors.

In continuing with FIG. 19, and to transform the new model into the linear system, the roots of the linear system must be determined (Block 256). More specifically, the first partial derivatives of the residual error, $E$, with respect to the shape descriptors, $\alpha_k$, are equal to zero. The error function, Equation 23, may be expressed in terms of the vertices, $v_i$, of the set, $V$, and the points, $p_i$, of the point cloud, Q:

$$E = \sum_{i=1}^{m} \lVert v_i - q_i \rVert^2 \qquad (24)$$

And may also be expressed in terms of the new model's shape descriptors as:

$$E = \left\| (V_{avg} + \sum_{k=1}^{W} a_k U_k') - Q \right\|^2 \qquad (25)$$

Where $V_{avg}$ is the set of vertices from the loaded model's vertices, which corresponds to the vertices set, $V$, that contains the closest vertices in the new model, $M_{new}$, that is being morphed to fit the bone point cloud, $Q$. $U_k'$ is a reduced version of the $k^{th}$ eigenbone, $U_k$, containing only the set of vertices corresponding to the vertices set, $V$.

Combining Equations 24 and 25, $E$ maybe expressed as:

$$E = \sum_{i=1}^{m} \left\| (v_{avg,i} + \sum_{k=1}^{W} a_k u_{k,i}') - q_i \right\|^2 \qquad (26)$$

Where $v_{avg,i}$ is the $i^{th}$ vertex of $V_{avg}$. Similarly, $u_{k,i}'$ is the $i^{th}$ vertex of the reduced eigenbone, $U_k'$.

The error function may be expanded as:

$$E = \sum_{i=1}^{m} \left[ \left( x_{avg,i} + \sum_{l=1}^{W} a_k x_{u',l,i} - x_{q,i} \right)^2 + \left( y_{avg,i} + \sum_{l=1}^{W} a_k y_{u',l,i} - y_{q,i} \right)^2 + \right.$$

$$\left( z_{avg,i} + \sum_{l=1}^{W} a_l z_{u',l,i} - z_{q,i} \right)^2 \right] \qquad (27)$$

Where $x_{avg,i}$ is the x-coordinate of the $i^{th}$ vertex of the average model, $x_{k,i}$ is the x-coordinate of the $i^{th}$ vertex of the $k^{th}$ eigenbone, and $x_{Q,i}$ is the x-coordinate of the $i^{th}$ point of the point cloud, $Q$. Similar arguments are applied to the y- and z-coordinates. Calculating the partial derivative of $E$ with respect to each shape descriptor, $\alpha_k$, yields:

$$\frac{\partial E}{\partial \alpha_k} = 0 \qquad \forall \quad k \, \epsilon \, [1, W] \qquad (28)$$

$$\frac{\partial E}{\partial \alpha_k} = \sum_{i=1}^{m} \left[ 2 \left( x_{avg,i} + \sum_{l=1}^{W} a_l x_{u',l,i} - x_{p,i} \right) x_{k,i} + 2 \left( y_{avg,i} + \sum_{l=1}^{W} a_l y_{u',l,i} - y_{p,i} \right) y_{k,i} \right.$$

$$\left. + 2 \left( z_{avg,i} + \sum_{l=1}^{W} a_k z_{u',l,i} - z_{p,i} \right) z_{k,i} \right] = 0 \, \forall \quad k \, \epsilon \, [1, W] \qquad (29)$$

Recombining the coordinate values into vectors yields:

$$\frac{\partial E}{\partial \alpha_k} = \sum_{i=1}^{m} \left[ (v_{avg,i} \cdot u_{k,i}') + \left( \sum_{l=1}^{W} a_l u_{l,i}' \right) \cdot u_{k,i}' - q_i \cdot u_{k,i}' \right]$$

$$= 0 \quad \forall \quad k \, \epsilon \, [1, W] \qquad (30)$$

And with rearrangement:

$$\sum_{i=1}^{m}\left(\sum_{l=1}^{w} a_l\left(u'_{l,i}\cdot u'_{k,i}\right)\right) = \sum_{i=1}^{m}\left[q_i\cdot u'_{k,i} - \left(v_{avg,i}\cdot u'_{k,i}\right)\right] \quad (31)$$

Reformulating Equation 31 into a matrix form provides a linear system of equations in the form of Ax = B:

$$\sum_{i=1}^{m}\begin{bmatrix} u'_{1,i}\cdot u'_{1,i} & u'_{2,i}\cdot u'_{1,i} & \cdots & \cdots & u'_{W,i}\cdot u'_{1,i} \\ u'_{1,i}\cdot u'_{2,i} & u'_{2,i}\cdot u'_{2,i} & \cdots & \cdots & u'_{W,i}\cdot u'_{2,i} \\ \vdots & \vdots & \ddots & \ddots & \vdots \\ \vdots & \vdots & \ddots & \ddots & \vdots \\ u'_{1,i}\cdot u'_{W,i} & u'_{2,i}\cdot u'_{W,i} & \cdots & \cdots & u'_{W,i}\cdot u'_{W,i} \end{bmatrix}\begin{bmatrix} a_1 \\ a_2 \\ \vdots \\ \vdots \\ a_W \end{bmatrix}$$

$$= \sum_{i=1}^{m}\begin{bmatrix} (q_i - v_{avg,i})\cdot u'_{1,i} \\ (q_i - v_{avg,i})\cdot u'_{2,i} \\ \vdots \\ \vdots \\ (q_i - v_{avg,i})\cdot u'_{W,i} \end{bmatrix} \quad (32)$$

The linear system of equations may be solved using any number of known methods, for instance, singular value decomposition (Block 258).

In one embodiment, the mahalanobis distance is omitted because the bone point clouds are dense, thus providing a constraining force on the model deformation. Therefore the constraining function of the mahalanobis distance may not be needed, but rather was avoided to provide the model deformation with more freedom to generate a new model that best fit the bone point cloud.

An ultrasound procedure in accordance with the embodiments of the present invention may, for example, generate approximately 5000 ultrasound images. The generated 3-D patient-specific models (Block 260, FIG. 7), when compared against CT-based segmented models, yielded an average error of approximately 2 mm.

The solution to the linear set of equations provides a description of the patient-specific 3-D model, derived from an average, or select model, from the statistical atlas, and optimized in accordance with the point cloud transformed from a bone contour that was isolated from a plurality of RF signals. The solution may be applied to the average model to display a patient-specific 3-D bone model for aiding in pre-operative planning, mapping out injection points, planning a physical therapy regiment, or other diagnostic and/or treatment-based procedure that involves a portion of the musculoskeletal system.

Cartilage 3-D models may be reconstructed a method that is similar to that which was outlined above for bone. During contour extraction, the contour of the cartilage is more difficult to detect than bone. Probabilistic modeling (Block 171) is used to process the raw RF signal to more easily identify cartilage, and SVM aids in detection of cartilage boundaries (Block 173) based on MRI training sets. A cartilage statistical atlas is formed by a method that may be similar to what was described for bone; however, as indicated previously, MRI is used rather than the CT (which was the case for bone). The segmentation (Block 216), variation extraction (Block 218) and base model morphing (Block 240) (FIG. 19) are processed to produce a reconstructed cartilage model in the same manner as a bone model is reconstructed. The cartilage model may be displayed alone, or in conjunction with the 3D patient-specific bone model.

Referring now to FIGS. 20-27, and in accordance with the invention, an additional method of extracting bone contours and generating point clouds from raw RF ultrasound signals is described. Referring now to FIG. 20, the ultrasound instrument 50 is shown in more detail with the electromagnetic tracking system 87, and the computer 54. The ultrasound instrument 50 may include an ultrasound transceiver 356 operatively coupled to the ultrasound probe 60 by a cable 66, and a controller 360. The ultrasound transceiver 356 generates drive signals that excite the ultrasound probe 60 so that the ultrasound probe 60 generates ultrasound signals 362 that can be transmitted into the patient. In an embodiment of the invention, the ultrasound signals 362 comprise bursts or pulses of ultrasound energy suitable for generating ultrasound images. The ultrasound probe 60 may also include the tracking marker 86, shown here as an electromagnetic tracking marker 86.

Reflected ultrasound signals, or echoes 364, are received by the ultrasound probe 60 and converted into RF signals that are transmitted to the transceiver 356. Each RF signal may be generated by a plurality of echoes 364, which may be isolated, partially overlapping, or fully overlapping. Each of the plurality of echoes 364 originates from a reflection of at least a portion of the ultrasound energy at an interface between two tissues having different densities, and represents a pulse-echo mode ultrasound signal. One type of pulse-echo mode ultrasound signal is known as an "A-mode" scan signal. The controller 360 converts the RF signals into a form suitable for transmission to the computer 54, such as by digitizing, amplifying, or otherwise processing the signals, and transmits the processed RF signals to the computer 54 via the I/O interface 85. The signals transmitted to the computer 54 may be raw RF signals representing the echoes 364 received by the ultrasound probe 60.

The electromagnetic tracking system 87 includes an electromagnetic transceiver unit 328 and an electromagnetic tracking system controller 366. The transceiver unit 328 may include one or more antennas 368, and transmits a first electromagnetic signal 370. The first electromagnetic signal 370 excites the tracking marker 86, which responds by transmitting a second electromagnetic signal 372 that is received by the transceiver unit 328. The tracking system controller 366 may then determine a relative position of the tracking marker 86 based on the received second electromagnetic signal 372. The tracking system controller 366 may then transmit tracking element position data to the computer 54 via I/O interface 85.

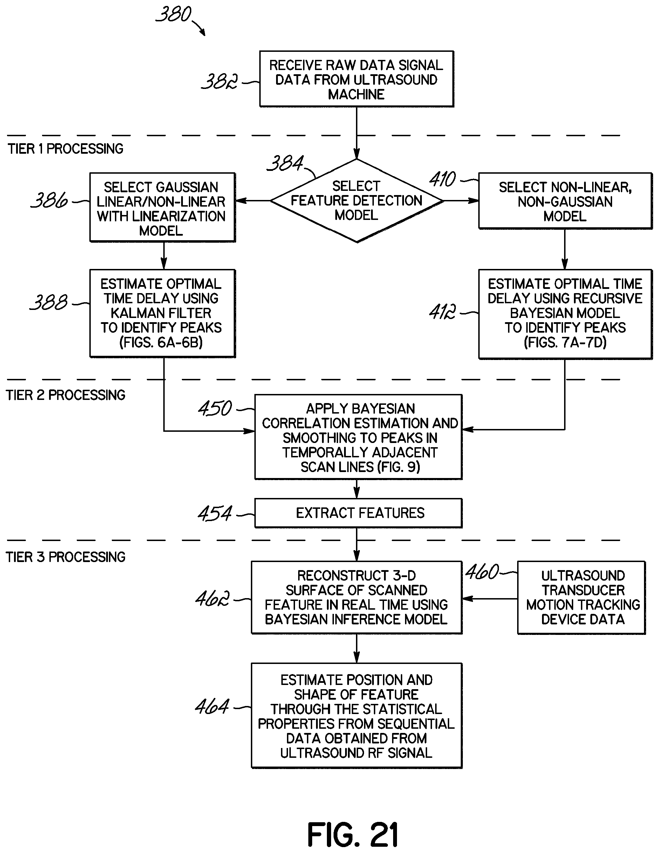

Referring now to FIG. 21, a flow chart 380 illustrates an alternative embodiment of the invention in which the acquired scan data is used to reconstruct patient-specific bone models. The patient-specific bone models may be generated from raw RF signals that are used directly to automatically extract bone contours from ultrasound scans. Specifically, these embodiments of the invention include additional methods of bone/cartilage contour detection, point cloud, and 3-D model reconstruction from ultrasound RF signal data. The ultrasound signal processing of these alternative embodiments optimizes scan reconstruction through a multi-tier signal processing model. The processing algorithm is broken down into multiple models, which are separated into different tiers. Each tier performs specific optimization or estimation to the data. The primary functions of the tiers include, but are not limited to, raw signal data optimization for features detection and estimation, scan-line features detection, global feature estimations, updates, and smoothing. The tiers operate within the framework of Bayesian inference model. The features and properties of the algorithm inputs are determined by mathematical and physical models within the tier. One example of this processing model implementation is a three-tier processing system, which is described below.

The first tier of the three-tier system optimizes the raw signal data and estimates the envelope of the feature vectors. The second tier estimates the features detected from each of the scan lines from the first tier, and constructs the parametric model for Bayesian smoothing. The third tier estimates the features extracted from the second tier to further estimate the three dimensional features in real-time using a Bayesian inference method.

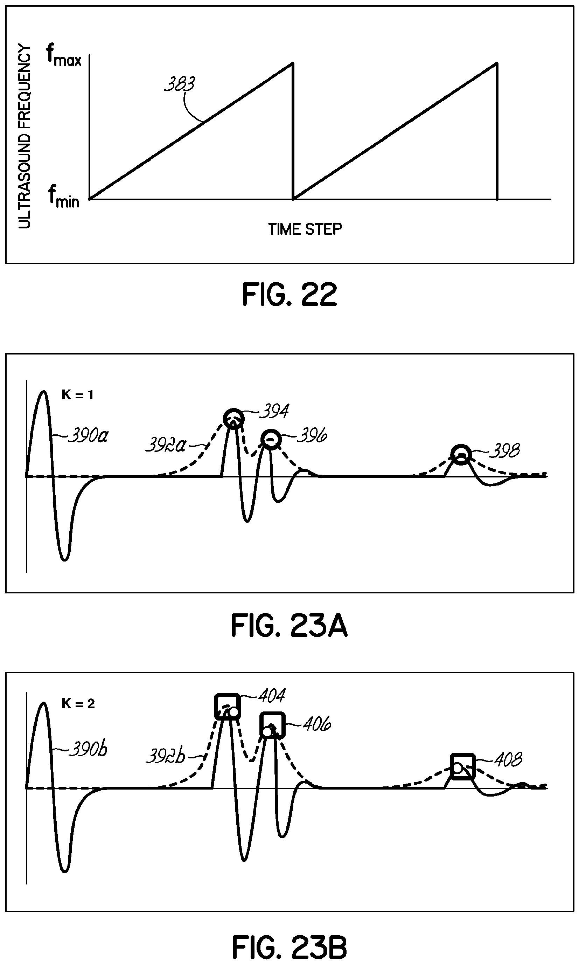

In block 382, raw RF signal data representing ultrasound echoes 364 detected by the ultrasound probe 60 is received by the program code 83 and processed by a first layer of filtering for feature detection. The feature vectors detected include bone, fat tissues, soft tissues, and muscles. The optimal outputs are envelopes of these features detected from the filter. There are two fundamental aspects of this design. The first aspect relates to the ultrasound probe 60 and the ultrasound controller firmware. In conventional ultrasound machines, the transmitted ultrasound signals 362 are generated at a fixed frequency during scanning. However, it has been determined that different ultrasound signal frequencies reveal different soft tissue features when used to scan the patient. Thus, according to the invention, the frequency of the transmitted ultrasound signal 362 changes with respect to time using a predetermined excitation funtion. One exemplary excitation function is a linear ramping sweep function 383, which is illustrated in FIG. 22.

The second aspect is to utilize data collected from multiple scans to support a Bayesian model for estimation, correction, and optimization. Two exemplary filter classes are illustrated in FIG. 21, either of which may be used to support the algorithm. In decision block 384, the program code 83 selects a feature detection model that determines the class of filter through which to process the RF signal data. If the data is to be processed by a linear filter, the application proceeds to block 386. In block 386, the imaging program code 83 selects a linear class of filter, such as a linear Gaussian model, or non-linear Gaussian model with linearization methods, based on the Kalman filter family. The operation of this linear class of filters is illustrated in more detail by FIGS. 23A and 23B, which outline the basic operation of the Kalman filter, upon which other extensions of the filter are built.

In block 388, an optimal time delay is estimated using a Kalman class filter to identify peaks in the amplitude or envelope of the RF signal. Referring now to FIG. 23A, at time k = 1, the filter is initialized by setting the ultrasound frequency $f_k = f_1$. The received echo or RF signal ($s_{obs}$) is represented by plot line 390a, while the signal envelope is represented by plot line 392a. The peak data matrix ($p_{k,fk}$), which contains the locations of the RF signal peaks, may be calculated by:

$$p_{k,fk} = E(s_{obs}) \quad (33)$$

where E is an envelope detection and extraction function. The peak data matrix ($p_{k,fk}$) thereby comprises a plurality of points representing the signal envelope 392, and can be used to predict the locations of envelope peaks 394, 396, 398 produced by frequency $f_{k+1}$ using the following equation:

$$p_{est,fk+1} = H(p_{k,fk+1}) \quad (34)$$

where H is the estimation function.

Referring now to FIG. 23B, at time k = 2, the filter enters a recursive portion of the algorithm. The frequency of the transmitted ultrasound signal 362 is increased so that $f_k = f_2$, and a new RF signal is received ($s_{obs,fk}$), as represented by plot line 390b. The new RF signal 390b also generates a new signal envelope 392b. A peak data matrix is calculated ($p_{k,fk}$) for the new signal envelope 392b, which identifies another set of peaks 404, 406, 408. The error of the prediction is computed by:

$$\varepsilon = p_{est,fk-1} - p_{k,fk} \quad (35)$$

and the error correction (Kalman) gain ($K_k$) is computed by:

$$K_k = P_k^- H^T (HP_k^- H^T + R) \quad (36)$$

where $P_{\overline{k}}$ is the error covariance matrix, and R is the covariance matrix of the measurement noise. The equation for estimating the peak data matrix for the next cycle becomes:

$$p_{est,k+1} = p_{k,fk} + K_k(\varepsilon) \quad (37)$$

and the error covariance is updated by:

$$P_k = (I - K_k H)P_k^- \quad (38)$$

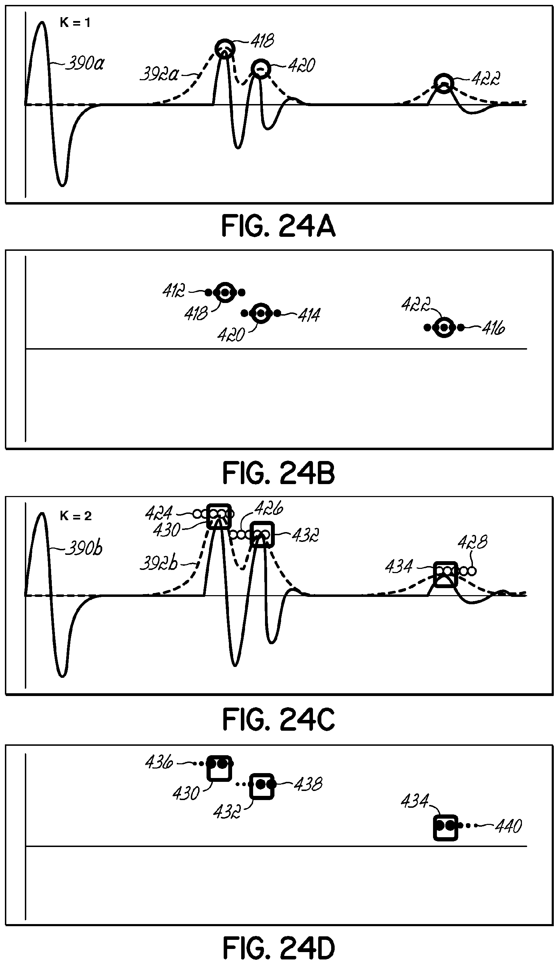

If the second class of filter is to be used, the program code 83 proceeds to block 410 rather than block 386 of flow chart 380, and selects a non-linear, non-Gaussian model that follows the recursive Bayesian filter approach. In the illustrated embodiment, a Sequential Monte Carlo method, or particles filter is shown as an exemplary implementation of the recursive Bayesian filter. In block 412, the program code 83 estimates an optimal time delay using the particles filter, to identify signal envelope peaks. An example of a particles filter is illustrated in FIGS. 24A and 24B. In principle, the particle filter generates a set of N equally weighted particles ($\rho_{k,fk}$) 412, 414, 416 around each envelope peak 418, 420, 422 of the peak data matrix detected during the initialization. The sets of equally weighted particles are based on an arbitrary statistical density ($\mathbb{P}$), which is approximated by:

$$\rho_{k,fk}^{i:1\rightarrow N} \sim \mathbb{P}(p_{k,fk}|s_{obs}) \quad (39)$$

These particles 411, 414, 416 predict the peak locations at $f_{k+1}$ via the following equation:

$$p_{est,fk+1}^{i:1\rightarrow N} = H(\rho_{k,fk}^{i:1\rightarrow N}) \quad (40)$$

where H is the estimation function.

Referring now to FIGS. 24C and 24D, at time k = 2, a new peak data matrix ($p_{k,fk}$) is calculated when the RF signal 90b ($s_{obs}$) becomes available, and new sets of estimation particles 424, 426, 428 are made around each peak 430, 432, 434 for ($f_k = f_2$). The estimation particles of sets 411, 414, 416 from time k =1 are compared with the observed data obtained at time k = 2, and an error is determined using the following equation:

$$\varepsilon_k^{i:1\rightarrow N} = p_{est,fk}^{i:1\rightarrow N} - p_{k,fk} \quad (41)$$

The normalized importance weights of the particles of particle sets 424, 426, 428 are evaluated as:

$$w_k^{i:1\to N} = \frac{\varepsilon_k^{i:1\to N}}{\Sigma_i^N \varepsilon_k^i} \quad (42)$$

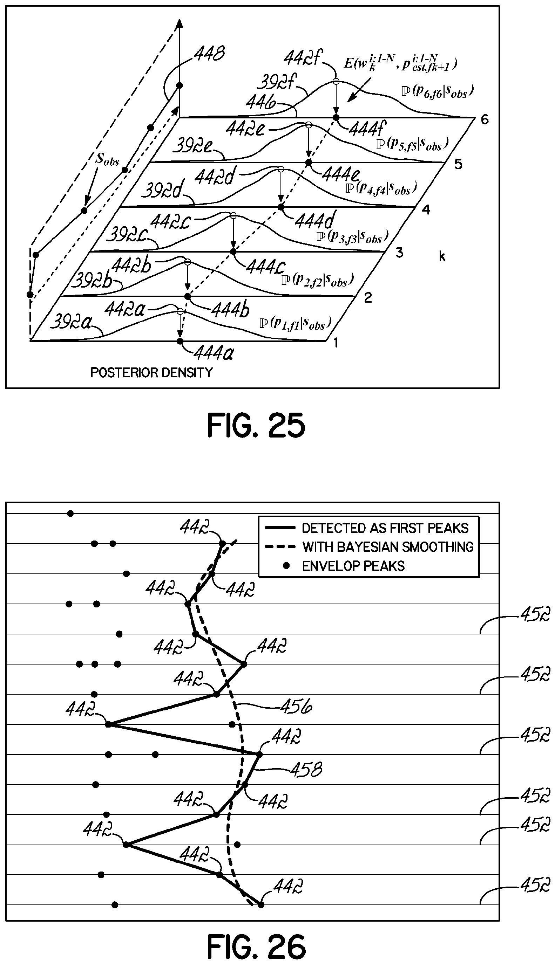

which produces weighted particle sets 436, 438, 440. This step is known as importance sampling where the algorithm approximates the true probability density of the system. An example of importance sampling is shown in FIG. 25, which illustrates a series of signal envelopes 392a-392f for times k = 1-6. Each signal envelope 392a-392f includes a peak 442a-442f and a projection 444a-444f of the peak 442a-442f onto a scan-line time scale 446 that indicates the echo return time. These projections 444a-444f may, in turn, be plotted as a contour 448 that represents an estimated location of a tissue density transition or surface. In any case, the expectation of the peak data matrix can then be calculated based on the importance weight and the particles' estimate:

$$p_{k,fk} = \mathbb{E}(w_k^{i:1\to N}, p_{est,fk+1}^{i:1\to N}) \quad (43)$$

In addition, particle maintenance may be required to avoid particle degeneracy, which refers to a result in which the weight is concentrated onto a few particles over time. Particle re-sampling can be used by replacing degenerated particles with new particles sampled from the posterior density:

$$\mathbb{P}(p_{est,fk+1}^{i:1\to N}) \quad (44)$$

Referring now to FIG. 26, once the envelope peaks have been identified, the program code 83 proceeds to block 450 and applies Bayesian smoothing to the envelope peaks 442 in temporally adjacent scan lines 452 before proceeding to block 454 and extracting 2-D features from the resulting smoothed contour line 456. This second layer of the filter thus applies a Bayesian technique to smooth the detected features on a two dimensional level. Conventional peak detection methods have a limitation in that the envelope peaks 442 across different scan lines are not statistically weighted. Thus, only the peaks 442 with the highest power are detected for reconstruction. This may result in an erroneous contour, as illustrated by contour line 458, which connects the envelope peaks 442 having the highest amplitude. Therefore, signal artifacts or improper amplitude compensation by gain control circuits in the RF signal path may obfuscate the signal envelope containing the feature of interest by distorting envelope peak amplitude. Hence, the goal of filtering in the second layer is to correlate signals from different scan lines to form a matrix that determines or identifies two-dimensional features.

This is achieved in embodiments of the invention by Bayesian model smoothing, which produces the exemplary smoothed contour line 456. The principle is to examine the signal envelope data retrospectively and attempt to reconstruct the previous state. The primarily difference between the Bayesian estimator and the smoother is that the estimator propagates the states forward in each recursive scan, while the smoother operates in the reverse direction. The initial state of the smoother begins at the last measurement and propagates backward. A common implementation of a smoother is the Rauch-Tung-Striebel (RTS) smoother. The feature embedded in the ultrasound signal is initialized based on a priori knowledge of the scan, which may include ultrasound transducer position data received from the electromagnetic tracking system 87. Sequential features are then estimated and updated in the ultrasound scan line with the RTS smoother.

In an embodiment of the invention, the ultrasound probe 60 is instrumented with the electromagnetic or optical tracking marker 86 so that the motion of the ultrasound probe 60 is accurately known. This tracking data 460 is provided to the program code 83 in block 462, and is needed to determine the position of the ultrasound probe 60 since the motion of the ultrasound probe 60 is arbitrary relative to the patient's joint. As scans are acquired by the ultrasound probe 60, the system estimates 3-D features of the joint, such as the shape of the bone and soft tissue. A tracking problem of this type can be viewed as a probability inference problem in which the objective is to calculate the most likely value of a state vector Xi given a sequence of measurements yi, which are the acquired scans. In an embodiment of the invention, the state vector Xi is the position of the ultrasound probe 60 with respect to some fixed known coordinate system or "world frame" (such as the ultrasound machine at time k=0), as well as the modes of the bone deformation. Two main steps in tracking are:

(1) Prediction - The states of the system at k=i can be predicted given all the measurements up through time k = i-1. To do this, the conditional probability $P(X_i \mid y_0, y_1, ..., y_{i-1})$, called the prior distribution, must be computed. If it is assumed that the process is a first order Markov process, this can be computed by integrating $P(X_i \mid X_{i-1})P(X_i \mid y_0, y_1, ..., y_{i-1})$ over all $X_{i-1}$.
and

(2) Correction - Given a new measurement $y_i$, correct the estimate of the state. To do this, the probability $P(X_i \mid y0, y1, ..., y_i)$, called the posterior distribution, must be computed.

A system dynamics model relates the previous state $X_{i-1}$ to the new state $X_i$ via the transitional distribution $P(X_i \mid X_{i-1})$, which is a model of how the state is expected to evolve with time. In an embodiment of the invention, $X_i$ are the 3-D feature estimates calculated from the Bayesian contour estimation performed during tier 2 filtering, and the transformation information contains the translations and rotations of the data obtained from the tracking system 87. With joint imaging, the optimal density or features are not expected to change over time, because the position of the bone is fixed in space and the shape of the bone scanned does not change. Hence, the transitional distribution does not alter the model states.

A measurement model relates the state to a predicted measurement, $y = f(X)$. Since there is uncertainty in the measurement, this relationship is generally expressed in terms of the conditional probability $P(y_i \mid X_i)$, also called the likelihood function. In an embodiment of the invention, the RF signal and a priori feature position and shape are related by an Anisotropic Iterative Closest Point (AICP) method.

To estimate position and shape of the feature, the program code 83 proceeds to block 464. At block 464, the program code 83 performs an AICP method that searches for the closest point between the two datasets iteratively to establish a correspondence by the anisotropic weighted distance that is calculated from the local error covariance of both datasets. The correspondence is then used to calculate a rigid transformation that is determined iteratively by minimizing the error until convergence. The 3-D features can then be predicted based on the received RF signal and the a priori feature position and shape. By calculating the residual error between the predicted 3-D feature and the RF signal data, the a priori position and shape of the feature are updated and corrected in each recursion. Using Bayes' rule, the posterior distribution can be computed based on measurements from the raw RF signal.

If both the dynamic model and the measurement model are linear with additive Gaussian noise, then the conditional probability distributions are normal distributions. In particular, $P(X_i \mid y_0, y_1, ..., y_i)$ is unimodal and Gaussian, and thus can be represented using the mean and covariance of the predicted measurements. Unfortunately, the measurement model is not linear and the likelihood function $P(y_i \mid X_i)$ is not Gaussian. One way to deal with this is to linearize the model about the local estimate, and assume that the distributions are locally Gaussian.

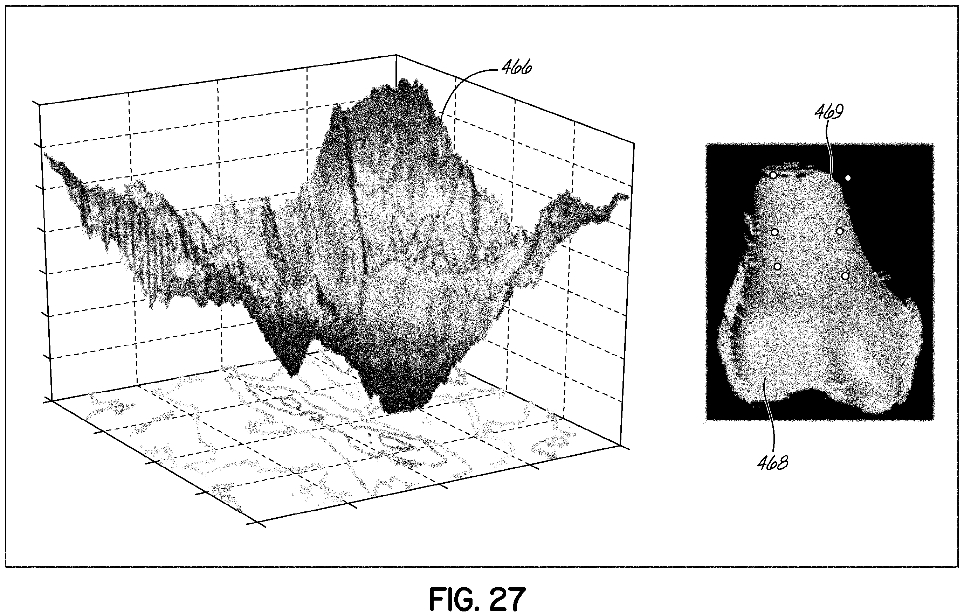

Referring to FIG. 27, a surface 466 representing an exemplary probability distribution associated with a point cloud 468 of a scanned bone 469 illustrates that the probability distribution for the measurement model is not Gaussian, and has many peaks. This suggests multiple hidden states are presented in the model. The posterior probability $P(X_i \mid y_0, y_1, ..., y_i)$ would also have multiple peaks. The problem would be worse if the state included shape parameters as well as position. A linear tracking filter such as the Kalman filter (or its nonlinear extension, the Extended Kalman filter) cannot deal with non-linear and non-Gaussian system with multi-peaks distribution, which may converge upon the wrong solution.

Instead of treating the probability distributions as Gaussian, a statistical inference can be performed using a Monte Carlo sampling of the states. The optimal position and shape of the feature are thereby estimated through the posterior density, which is determined from sequential data obtained from the RF signals. For recursive Bayesian estimation, one exemplary implementation is particle filtering, which has been found to be useful in dealing in applications where the state vector is complex and the data contain a great deal of clutter, such as tracking objects in image sequences. The basic idea is to represent the posterior probability by a set of independent and identically distributed weighted samplings of the states, or particles. Given enough samples, even very complex probability distributions can be represented. As measurements are taken, the importance weights of the particles are adjusted using the likelihood model, using the equation $w_j' = P(y_i \mid X_i) w_j$, where $w_j$ is the weight of the j-th particle. This is known as importance sampling.

The principal advantage of this method is that the method can approximate the true probability distribution of the system, which cannot be determined directly, by approximating a finite set of particles from a distribution from which samples can be drawn. As measurements are obtained, the algorithm adjusts the particle weights to minimize the error between the prediction and observation states. With enough particles and iterations, the posterior distribution will approach the true density of the system. A plurality of bone or other anatomical feature surface contour lines is thereby generated that can be used to generate 3-D images and models of the joint or anatomical feature. These models, in turn, may be used to facilitate medical procedures, such as joint injections, by allowing the joint or other anatomical feature to be visualized in real time during the procedure using an ultrasound scan.

While the present invention has been illustrated by the description of the embodiments thereof, and while the embodiments have been described in considerable detail, it is not the intention of the applicant to restrict or in any way limit the scope of the appended claims to such detail.

**Claims**

1.  A computer implemented method of generating a 3-D patient-specific musculoskeletal model to be executed on an ultrasound medical imaging system, the method comprising:

    transmitting ultrasound signals (362) the frequency of which changes with respect to time using a predetermined excitation function (383) as part of an ultrasound scan;
    acquiring a plurality of raw radio frequency (RF) signals (364) from the ultrasound scan, the plurality of raw RF signals (364) including a first raw RF signal (390a) and a second raw RF signal (390b), each raw RF signal representing a reflected ultrasound signal;
    generating an envelope signal (392a, 392b) from each of the raw RF signals (390a, 390b), each envelope signal (392a, 392b) including one or more peaks (442a, 442b);
    generating a contour line (456) based on the peaks (442a, 442b) of the envelope signals (392a, 392b);
    determining a position of the ultrasound transducer (68) corresponding to each of the plurality of raw RF signals (390a, 390b);
    transforming the contour line into a point cloud (468) based at least in part on the positions of the ultrasound transducer (68); and
    optimizing a 3-D bone model with respect to the point cloud (468) to generate the 3-D patient-specific musculoskeletal model.

2.  The method according to claim 1 wherein the first raw RF signal (390a) is at a first frequency and the second raw RF signal (390b) is at a second frequency different from the first frequency.

3.  The method according to either claim 1 or claim 2 wherein:

    transmitting ultrasound signals comprises:

    transmitting a plurality of ultrasound pulses each having a frequency; and,
    changing the frequency of the ultrasound pulses as the raw RF signals (364) are acquired;

    acquiring the plurality of raw RF signals comprises:

    receiving reflected ultrasound energy from each pulse; and,
    converting the reflected ultrasound energy from each pulse into the raw RF signals (364).

4.  The method according to claim 3 wherein the frequency of the ultrasound pulses is changed by sweeping the frequency.

5.  The method according to any one of claims 1-4 wherein generating the contour line (456) from the envelope signals (392a, 392b) comprises:

    identifying a first peak (442a) of the one or more peaks (442a-f) in each of the envelope signals (392a, 392b); and
    generating the contour line (456) by applying a first filter to the first peaks (442a) in temporally adjacent envelope signals (452).

6.  The method according to claim 5 wherein the first peak (442a) is identified in each of the envelope signals (392a, 392b) by using a second filter to estimate an optimal time delay.

7.  The method according to claim 6 wherein the second filter is selected from a group consisting of a Kalman filter, a recursive Bayesian filter, and a particles filter.

8.  The method according to any one of claims 5-7 wherein the first filter is based on Bayesian model smoothing.

9.  The method according to any one of claims 1-5 wherein generating the contour line (456) from the plurality of raw RF signals (364) comprises:

    calculating a first peak data matrix for the envelope signal (392a) of the first raw RF signal (390a);
    estimating a second peak data matrix for the envelope signal (392b) of the second raw RF signal (390b) based on the first peak data matrix;
calculating the second peak data matrix from the envelope signal (392b) of the second raw RF signal;
calculating an error matrix based on a difference between the estimated second peak data matrix and the calculated second peak data matrix; and
calculating an error correction gain based on the error matrix.

10. The method according to claim 9 comprising:
estimating a third peak data matrix for the envelope signal of a third raw RF signal by multiplying the error matrix by the error correction gain, and adding a product of the multiplication to the calculated second peak data matrix.

11. The method according to any one of claims 1-5 wherein generating the contour line (456) from the plurality of raw RF signals (364) comprises:

calculating a first peak data matrix for the envelope signal (392a) of the first raw RF signal (390a);
generating a first set of particles (412) for each peak (418) in the first peak data matrix;
estimating a second peak data matrix for the envelope signal (392b) of the second raw RF signal (390b) based on the first set of particles (412) for each peak (418) in the first peak data matrix;
calculating a second peak data matrix for the envelope signal (392b) of the second raw RF signal (390b);
calculating an error matrix based on a difference between the estimated second peak data matrix and the calculated second peak data matrix;
generating a second set of particles (424) for each peak (430) in the second peak data matrix; and
calculating a weight for each particle in the second set of particles (424) for each peak (430) in the second peak data matrix based on the error matrix.

12. The method according to claim 11 comprising:
estimating a third peak data matrix for an envelope signal of a third raw RF signal (390c) based on the weighted particles of the second set of particles for each peak in the second peak data matrix.

13. The method according to claim 10 or 12 wherein the first raw RF signal (390a), the second raw RF signal (390b), and the third raw RF signal (390c) are temporally adjacent raw RF signals.

14. The method according to any one of claims 1-13 comprising: extracting a 2-D feature from the contour line (456).

15. The method according to any one of claims 1-14 wherein the contour line (448) is generated by:

applying a statistical weight to each of the one or more peaks (442a, 442b) in each of the envelope signals (392a, 392b); and
determining a path of the contour line (456) across the envelope signals (392a, 392b) based on the weighted peaks.

16. An ultrasound medical imaging system (50) for generating a 3-D patient-specific musculoskeletal model, the apparatus (50) comprising:

a processor (54); and
a memory (81) containing instructions that, when executed by the processor (54), cause the apparatus (50) to:

transmit ultrasound signals (362) the frequency of which change with respect to time using a predetermined excitation function (383) as part of an ultrasound scan;
acquire a plurality of raw radio frequency (RF) signals (364) from the ultrasound scan, the plurality of raw RF signals (364) including a first raw RF signal (390a) and a second raw RF signal (390b), each raw RF signal representing a reflected ultrasound signal;
generate an envelope signal (392a, 392b) from each of the raw RF signals (390a, 390b), each envelope signal (392a, 392b) including one or more peaks (442a, 442b);
generate a contour line (456) based on the peaks (442a, 442b) of the envelope signals (392a, 392b);
determining a position of the ultrasound transducer (68) corresponding to each of the plurality of raw RF signals (390a, 390b);
transforming the contour line into a point cloud (468) based at least in part on the positions of the ultrasound transducer (68); and

optimizing a 3-D bone model with respect to the point cloud (468) to generate the 3-D patient-specific musculoskeletal model.

**Patentansprüche**

1.  Computerimplementiertes Verfahren zum Erzeugen eines patientenspezifischen 3D-Bewegungsapparatmodells, das auf einem medizinischen Ultraschallbildgebungssystem auszuführen ist, wobei das Verfahren umfasst:

    Senden von Ultraschallsignalen (362), deren Frequenz sich in Bezug auf die Zeit ändert, unter Verwendung einer vorbestimmten Anregungsfunktion (383) als Teil eines Ultraschallscans;
    Erfassen einer Mehrzahl von Hochfrequenz(RF)-Rohsignalen (364) aus dem Ultraschallscan, wobei die Mehrzahl von RF-Rohsignalen (364) ein erstes RF-Rohsignal (390a) und ein zweites RF-Rohsignal (390b) einschließt, wobei jedes RF-Rohsignal ein reflektiertes Ultraschallsignal repräsentiert;
    Erzeugen eines Hüllkurvensignals (392a, 392b) aus jedem der RF-Rohsignale (390a, 390b), wobei jedes Hüllkurvensignal (392a, 392b) eine oder mehrere Spitzen (442a, 442b) einschließt;
    Erzeugen einer Konturlinie (456) basierend auf den Spitzen (442a, 442b) der Hüllkurvensignale (392a, 392b);
    Bestimmen einer Position der Ultraschallsonde (68), die jedem der Mehrzahl von RF-Rohsignalen (390a, 390b) entspricht;
    Transformieren der Konturlinie in eine Punktwolke (468) basierend zumindest teilweise auf den Positionen der Ultraschallsonde (68); und
    Optimieren eines 3D-Knochenmodells in Bezug auf die Punktwolke (468), um das patientenspezifische 3D-Bewegungsapparatmodell zu erzeugen.

2.  Verfahren nach Anspruch 1, wobei das erste RF-Rohsignal (390a) auf einer ersten Frequenz liegt und das zweite RF-Rohsignal (390b) auf einer sich von der ersten Frequenz unterscheidenden zweiten Frequenz liegt.

3.  Verfahren nach entweder Anspruch 1 oder Anspruch 2, wobei das Senden der Ultraschallsignale umfasst:

    Senden einer Mehrzahl von Ultraschallimpulsen, von denen jeder eine Frequenz aufweist; und
    Ändern der Frequenz der Ultraschallimpulse, wenn die RF-Rohsignale (364) erfasst werden;

    das Erfassen der Mehrzahl von RF-Rohsignalen umfasst:

    Empfangen einer reflektierten Ultraschallenergie von jedem Impuls; und
    Umwandeln der reflektierten Ultraschallenergie von jedem Impuls in die RF-Rohsignale (364).

4.  Verfahren nach Anspruch 3, wobei die Frequenz der Ultraschallimpulse durch Sweeping der Frequenz geändert wird.

5.  Verfahren nach einem der Ansprüche 1 bis 4, wobei das Erzeugen der Konturlinie (456) aus den Hüllkurvensignalen (392a, 392b) umfasst:

    Identifizieren einer ersten Spitze (442a) der einen oder der mehreren Spitzen (442a-f) in jedem der Hüllkurvensignale (392a, 392b); und
    Erzeugen der Konturlinie (456) durch Anwenden eines ersten Filters auf die ersten Spitzen (442a) in zeitlich benachbarten Hüllkurvensignalen (452).

6.  Verfahren nach Anspruch 5, wobei die erste Spitze (442a) in jedem der Hüllkurvensignale (392a, 392b) unter Verwendung eines zweiten Filters identifiziert wird, um eine optimale Zeitverzögerung zu schätzen.

7.  Verfahren nach Anspruch 6, wobei der zweite Filter aus einer Gruppe ausgewählt ist, die aus einem Kalman-Filter, einem rekursiven Bayesschen Filter und einem Partikelfilter besteht.

8.  Verfahren nach einem der Ansprüche 5 bis 7, wobei der erste Filter auf Bayesscher Modellglättung basiert.

9.  Verfahren nach einem der Ansprüche 1 bis 5, wobei das Erzeugen der Konturlinie (456) aus der Mehrzahl von RF-Rohsignalen (364) umfasst:

Berechnen einer ersten Spitzendatenmatrix für das Hüllkurvensignal (392a) des ersten RF-Rohsignals (390a);
Schätzen einer zweiten Spitzendatenmatrix für das Hüllkurvensignal (392b) des zweiten RF-Rohsignals (390b) basierend auf der ersten Spitzendatenmatrix;
Berechnen der zweiten Spitzendatenmatrix aus dem Hüllkurvensignal (392b) des zweiten RF-Rohsignals;
Berechnen einer Fehlermatrix basierend auf einem Unterschied zwischen der geschätzten zweiten Spitzendatenmatrix und der berechneten zweiten Spitzendatenmatrix; und
Berechnen einer Fehlerkorrekturverstärkung basierend auf der Fehlermatrix.

10. Verfahren nach Anspruch 9, umfassend:
Schätzen einer dritten Spitzendatenmatrix für das Hüllkurvensignal eines dritten RF-Rohsignals durch Multiplizieren der Fehlermatrix mit der Fehlerkorrekturverstärkung und Addieren eines Produkts der Multiplikation zu der berechneten zweiten Spitzendatenmatrix.

11. Verfahren nach einem der Ansprüche 1 bis 5, wobei das Erzeugen der Konturlinie (456) aus der Mehrzahl von RF-Rohsignalen (364) umfasst:

Berechnen einer ersten Spitzendatenmatrix für das Hüllkurvensignal (392a) des ersten RF-Rohsignals (390a);
Erzeugen eines ersten Satzes von Partikeln (412) für jede Spitze (418) in der ersten Spitzendatenmatrix;
Schätzen einer zweiten Spitzendatenmatrix für das Hüllkurvensignal (392b) des zweiten RF-Rohsignals (390b) basierend auf dem ersten Satz von Partikeln (412) für jede Spitze (418) in der ersten Spitzendatenmatrix;
Berechnen einer zweiten Spitzendatenmatrix für das Hüllkurvensignal (392b) des zweiten RF-Rohsignals (390b);
Berechnen einer Fehlermatrix basierend auf einem Unterschied zwischen der geschätzten zweiten Spitzendatenmatrix und der berechneten zweiten Spitzendatenmatrix;
Erzeugen eines zweiten Satzes von Partikeln (424) für jede Spitze (430) in der zweiten Spitzendatenmatrix; und
Berechnen einer Gewichtung für jeden Partikel in dem zweiten Satz von Partikeln (424) für jede Spitze (430) in der zweiten Spitzendatenmatrix basierend auf der Fehlermatrix.

12. Verfahren nach Anspruch 11, umfassend:
Schätzen einer dritten Spitzendatenmatrix für ein Hüllkurvensignal eines dritten RF-Rohsignals (390c) basierend auf den gewichteten Partikeln des zweiten Satzes von Partikeln für jede Spitze in der zweiten Spitzendatenmatrix.

13. Verfahren nach Anspruch 10 oder 12, wobei das erste RF-Rohsignal (390a), das zweite RF-Rohsignal (390b) und das dritte RF-Rohsignal (390c) zeitlich benachbarte RF-Rohsignale sind.

14. Verfahren nach einem der Ansprüche 1 bis 13, umfassend:
Extrahieren eines 2D-Merkmals aus der Konturlinie (456).

15. Verfahren nach einem der Ansprüche 1 bis 14, wobei die Konturlinie (448) erzeugt wird durch:

Anwenden einer statistischen Gewichtung auf jede der einen oder der mehreren Spitzen (442a, 442b) in jedem der Hüllkurvensignale (392a, 392b); und
Bestimmen eines Pfades der Konturlinie (456) über die Hüllkurvensignale (392a, 392b) basierend auf den gewichteten Spitzen.

16. Medizinisches Ultraschallbildgebungssystem (50) zum Erzeugen eines patientenspezifischen 3D-Bewegungsapparatmodells, wobei die Vorrichtung (50) umfasst:

einen Prozessor (54); und
einen Speicher (81), der Anweisungen enthält, die, wenn sie von dem Prozessor (54) ausgeführt werden, bewirken, dass die Vorrichtung (50):

Ultraschallsignale (362), deren Frequenz sich in Bezug auf die Zeit ändert, unter Verwendung einer vorbestimmten Anregungsfunktion (383) als Teil eines Ultraschallscans sendet;
eine Mehrzahl von Hochfrequenz(RF)-Rohsignalen (364) aus dem Ultraschallscan erfasst, wobei die Mehrzahl von RF-Rohsignalen (364) ein erstes RF-Rohsignal (390a) und ein zweites RF-Rohsignal (390b) einschließt, wobei jedes RF-Rohsignal ein reflektiertes Ultraschallsignal repräsentiert;
ein Hüllkurvensignal (392a, 392b) aus jedem der RF-Rohsignale (390a, 390b) erzeugt, wobei jedes Hüllkurvensignal (392a, 392b) eine oder mehrere Spitzen (442a, 442b) einschließt;

eine Konturlinie (456) basierend auf den Spitzen (442a, 442b) der Hüllkurvensignale (392a, 392b) erzeugt;

eine Position der Ultraschallsonde (68), die jedem der Mehrzahl von RF-Rohsignalen (390a, 390b) entspricht, bestimmt;

die Konturlinie basierend zumindest teilweise auf den Positionen der Ultraschallsonde (68) in eine Punktwolke (468) transformiert; und

ein 3D-Knochenmodell in Bezug auf die Punktwolke (468) optimiert, um das patientenspezifische 3D-Bewegungsapparatmodell zu erzeugen.

**Revendications**

1. Procédé informatique de génération d'un modèle musculosquelettique en 3D spécifique à un patient à exécuter sur un système d'imagerie médicale par ultrasons, le procédé comprenant :

la transmission de signaux ultrasonores (362) dont la fréquence change en fonction du temps à l'aide d'une fonction d'excitation prédéterminée (383) comme une partie d'une échographie ;

l'acquisition d'une pluralité de signaux radio fréquence (RF) bruts (364) de l'échographie, la pluralité de signaux RF bruts (364) incluant un premier signal RF brut (390a) et un deuxième signal RF brut (390b), chaque signal RF brut représentant un signal ultrasonore réfléchi ;

la génération d'un signal d'enveloppe (392a, 392b) à partir de chacun des signaux RF bruts (390a, 390b), chaque signal d'enveloppe (392a, 392b) incluant une ou plusieurs crêtes (442a, 442b) ;

la génération d'une ligne de contour (456) sur la base des crêtes (442a, 442b) des signaux d'enveloppe (392a, 392b) ;

la détermination d'une position du transducteur à ultrasons (68) correspondant à chacun de la pluralité de signaux RF bruts (390a, 390b) ;

la transformation de la ligne de contour en un nuage de points (468) au moins partiellement sur la base des positions du transducteur à ultrasons (68) ; et

l'optimisation d'un modèle osseux en 3D par rapport au nuage de points (468) pour générer un modèle musculosquelettique en 3D spécifique à un patient.

2. Procédé selon la revendication 1 dans lequel le premier signal RF brut (390a) est à une première fréquence et le second signal RF brut (390b) est à une deuxième fréquence différente de la première fréquence.

3. Procédé selon l'une ou l'autre de la revendication 1 ou de la revendication 2 dans lequel :

la transmission de signaux ultrasonores comprend :

la transmission d'une pluralité d'impulsions ultrasonores ayant chacune une fréquence ; et

le changement de la fréquence des impulsions ultrasonores alors que les signaux RF bruts (364) sont acquis ;

l'acquisition de la pluralité de signaux RF bruts comprend :

la réception d'une énergie ultrasonore réfléchie de chaque impulsion ; et

la conversion de l'énergie ultrasonore réfléchie de chaque impulsion dans les signaux RF bruts (364).

4. Procédé selon la revendication 3 dans lequel la fréquence des impulsions ultrasonores est modifiée par balayage de la fréquence.

5. Procédé selon l'une quelconque des revendications 1 à 4 dans lequel la génération de la ligne de contour (456) à partir des signaux d'enveloppe (392a, 392b) comprend :

l'identification d'une première crête (442a) des une ou plusieurs crêtes (442a-f) dans chacun des signaux d'enveloppe (392a, 392b) ; et

la génération d'une ligne de contour (456) par application d'un premier filtre à la première crête (442a) dans des signaux d'enveloppe temporairement adjacents (452).

**6.** Procédé selon la revendication 5 dans lequel la première crête (442a) est identifiée dans chacun des signaux d'enveloppe (392a, 392b) en utilisant un second filtre pour estimer un délai optimal.

**7.** Procédé selon la revendication 6 dans lequel le second filtre est sélectionné à partir d'un groupe consistant en un filtre de Kalman, un filtre bayésien récursif, et un filtre à particules.

**8.** Procédé selon l'une quelconque des revendications 5 à 7 dans lequel le premier filtre se base sur un lissage de modèle bayésien.

**9.** Procédé selon l'une quelconque des revendications 1 à 5 dans lequel la génération de la ligne de contour (456) à partir de la pluralité de signaux RF bruts (364) comprend :

le calcul d'une première matrice de données de crête pour le signal d'enveloppe (392a) du premier signal RF brut (390a) ;
l'estimation d'une deuxième matrice de données de crête pour le signal d'enveloppe (392b) du deuxième signal RF brut (390b) sur la base de la première matrice de données de crête ;
le calcul de la deuxième matrice de données de crête à partir du signal d'enveloppe (392b) du deuxième signal RF brut ;
le calcul d'une matrice d'erreur sur la base d'une différence entre la deuxième matrice de données de crête estimée et la deuxième matrice de données de crête calculée ; et
le calcul d'un gain de correction d'erreur sur la base de la matrice d'erreur.

**10.** Procédé selon la revendication 9 comprenant :
l'estimation d'une troisième matrice de données de crête pour le signal d'enveloppe d'un troisième signal RF brut par multiplication de la matrice d'erreur par le gain de correction d'erreur, et ajout d'un produit de la multiplication à la deuxième matrice de données de crête calculée.

**11.** Procédé selon l'une quelconque des revendications 1 à 5 dans lequel la génération de la ligne de contour (456) à partir de la pluralité de signaux RF bruts (364) comprend :

le calcul d'une première matrice de données de crête pour le signal d'enveloppe (392a) du premier signal RF brut (390a) ;
la génération d'un premier ensemble de particules (412) pour chaque crête (418) dans la première matrice de données de crête ;
l'estimation d'une deuxième matrice de données de crête pour le signal d'enveloppe (392b) du deuxième signal RF brut (390b) sur la base du premier ensemble de particules (412) pour chaque crête (418) dans la première matrice de données de crête ;
le calcul d'une deuxième matrice de données de crête pour le signal d'enveloppe (392b) du deuxième signal RF brut (390b) ;
le calcul d'une matrice d'erreur sur la base d'une différence entre la deuxième matrice de données de crête estimée et la deuxième matrice de données de crête calculée ;
la génération d'un second ensemble de particules (424) pour chaque crête (430) dans la deuxième matrice de données de crête ; et
le calcul d'une pondération pour chaque particule dans le deuxième ensemble de particules (424) pour chaque crête (430) dans la deuxième matrice de données de crête sur la base de la matrice d'erreur.

**12.** Procédé selon la revendication 11 comprenant :
l'estimation d'une troisième matrice de données de crête pour un signal d'enveloppe d'un troisième signal RF brut (390c) sur la base des particules pondérées du deuxième ensemble de particules pour chaque crête dans la deuxième matrice de données de crête.

**13.** Procédé selon la revendication 10 ou 12, dans lequel le premier signal RF brut (390a), le deuxième signal RF brut (390b), et le troisième signal RF brut (390c) sont des signaux RF bruts temporairement adjacents.

**14.** Procédé selon l'une quelconque des revendications 1 à 13 comprenant :
l'extraction d'un élément en 2D de la ligne de contour (456).

**15.** Procédé selon l'une quelconque des revendications 1 à 14 dans lequel la ligne de contour (448) est générée par :

application d'une pondération statistique à chacune des une ou plusieurs crêtes (442a, 442b) dans chacun des signaux d'enveloppe (392a, 392b) ; et

détermination d'une trajectoire de la ligne de contour (456) à travers les signaux d'enveloppe (392a, 392b) sur la base des crêtes pondérées.

16. Système d'imagerie médicale par ultrasons (50) pour générer un modèle musculosquelettique en 3D spécifique à un patient, l'appareil (50) comprenant :

un processeur (54) ; et

une mémoire (81) contenant des instructions qui, lorsqu'elles sont exécutées par le processeur (54), amènent l'appareil (50) à :

transmettre des signaux ultrasonores (362) dont la fréquence change en fonction du temps à l'aide d'une fonction d'excitation prédéterminée (383) comme une partie d'une échographie ;

acquérir une pluralité de signaux radio fréquence (RF) bruts (364) de l'échographie, la pluralité de signaux RF bruts (364) incluant un premier signal RF brut (390a) et un deuxième signal RF brut (390b), chaque signal RF brut représentant un signal ultrasonore réfléchi ;

générer un signal d'enveloppe (392a, 392b) à partir de chacun des signaux RF bruts (390a, 390b), chaque signal d'enveloppe (392a, 392b) incluant une ou plusieurs crêtes (442a, 442b) ;

générer une ligne de contour (456) sur la base des crêtes (442a, 442b) des signaux d'enveloppe (392a, 392b) ;

déterminer une position du transducteur à ultrasons (68) correspondant à chacun de la pluralité de signaux RF bruts (390a, 390b) ;

transformer la ligne de contour en un nuage de points (468) au moins partiellement sur la base des positions du transducteur à ultrasons (68) ; et

optimiser un modèle osseux en 3D par rapport au nuage de points (468) pour générer un modèle musculosquelettique en 3D spécifique à un patient.

**FIG. 1**

FIG. 2

FIG. 2A

FIG. 3

DETERMINE A PLURALITY OF CALIBRATION PARAMETERS OF THE ULTRASOUND PROBE WITH RESPECT TO THE LOCAL FRAME OF REFERENCE — 102

HOLD THE HYBRID PROBE STATIONARY WITH RESPECT TO THE POSITION SENSOR AND ACQUIRE A NUMBER OF POSITION POINTS — 104

RECORD $T_{OP}^{W}$ — 106

CALCULATE THE PLURALITY OF CALIBRATION PARAMETERS AND $T_{W}^{OP}$ — 108

END

FIG. 4

FIG. 5A          FIG. 5B          FIG. 5C

FIG. 6A

FIG. 6B

FIG. 6C

FIG. 6D

FIG. 6E

FIG. 6F

*150*

POSITION PATIENT'S KNEE JOINT IN A FIRST DEGREE OF FLEXION AND ACQUIRE A-MODE RF SIGNAL —*152*

ACQUIRE B-MODE IMAGE —*154*

POSITION PATIENT'S KNEE JOINT IN ANOTHER DEGREE OF FLEXION AND ACQUIRE A-MODE RF SIGNAL —*156*

ACQUIRE B-MODE IMAGE —*158*

*160* ACQUISITION COMPLETE ? — N / Y

EXTRACT BONE CONTOUR FROM EACH RF SIGNAL ACQUIRED —*164*

GENERATE A BONE POINT CLOUD —*186*

*210* BONE MODEL ? — SELECTED / AVERAGE

*232* LOAD BONE MODEL FROM STATISTICAL ATLAS BASED ON DEMOGRAPHICS OF PATIENT

LOAD AVERAGE BONE MODEL FROM STATISTICAL ATLAS —*230*

APPLY BONE POINT CLOUD TO THE LOADED MODEL —*234*

*236* CONSTRAIN ONE OR MORE SHAPE DESCRIPTORS ? — Y / N

*238* SET CONSTRAINTS

OPTIMIZE SHAPE DESCRIPTORS OF LOADED MODEL WITH RESPECT TO BONE POINT CLOUD —*240*

OUTPUT 3D PATIENT-SPECIFIC MODEL —*260*

END

FIG. 7

FIG. 8

FIG. 9

**FIG. 10A**

**FIG. 10B**

**FIG. 10C**

FIG. 10D

FIG. 1OE

OFFLINE PARAMETERS (i.e. ATTENUATION COEFFICIENT, ACOUSTICAL IMPEDANCE, ETC.) —169

164

APPLY MODEL-BASED SIGNAL PROCESSING —167

APPLY MOVING POWER FILTER TO EACH ACQUIRED RF SIGNAL TO PRODUCE A PLURALITY OF ENVELOPES —168

171

PROBABILISTIC MODEL

IDENTIFY THE BONE ECHO IN EACH OF THE PLURALITY OF ENVELOPES —170

EXTRACT THE BONE ECHOES FROM THE PLURALITY OF ENVELOPES —172

DETECT CARTILAGE —173

GENERATE A BONE CONTOUR FROM THE EXTRACTED ECHOES —174

REMOVE ISOLATED OUTLIERS —176

REMOVE FALSE BONE ECHOES —178

END

FIG. 11

186

TRANSFORM RESULTANT BONE CONTOURS FROM ULTRASOUND FRAME TO WORLD FRAME TO GENERATE POINT CLOUDS —188

REGISTER RESULTANT BONE CONTOUR WITH ASSOCIATED B-MODE IMAGE —190

ALIGN WHOLE BONE POINT CLOUD TO MODEL OF THE BONE —192

INTEGRATE PLURALITY OF POINT CLOUDS INTO A WHOLE BONE POINT CLOUD —202

END

FIG. 15

FIG. 12A

FIG. 12B

FIG. 12C

FIG. 12D

FIG. 12E

**FIG. 13A**

**FIG. 13D**

**FIG. 13B**

**FIG. 13E**

**FIG. 13C**

**FIG. 13F**

**FIG. 14A**

**FIG. 14D**

**FIG. 14B**

**FIG. 14E**

**FIG. 14C**

**FIG. 14F**

FIG. 16B

FIG. 16A

FIG. 16C

FIG. 16D

EP 2 950 712 B1

FIG. 17A

FIG. 17B

EP 2 950 712 B1

**FIG. 17C**

**FIG. 17D**

EP 2 950 712 B1

*212*

```
┌─────────────────────────────────────────────────┐
│   ACQUIRE CT IMAGE OF EACH BONE FOR THE DATASET   │── 214
└─────────────────────────────────────────────────┘
                        │
                        ▼
┌─────────────────────────────────────────────────┐
│ SEGMENT CT IMAGES AND CONSTRUCT 3D MODEL FOR EACH │── 216
│               BONE IN THE DATASET                 │
└─────────────────────────────────────────────────┘
                        │
                        ▼
┌─────────────────────────────────────────────────┐
│         EXTRACT VARIATIONS IN EACH 3D MODEL       │── 218
│             COMPRISING THE DATASET                │
└─────────────────────────────────────────────────┘
                        │
                        ▼
┌─────────────────────────────────────────────────┐
│       EXTRACT AN AVERAGE MODEL FROM ALL 3D        │── 220
│          MODELS COMPRISING THE DATASET            │
└─────────────────────────────────────────────────┘
                        │
                        ▼
                    ( END )
```

## FIG. 18

*240*

```
┌─────────────────────────────────────────────────┐
│ SEARCH SHAPE DESCRIPTORS OF LOADED MODEL FOR BEST │── 250
│         MATCH TO WHOLE BONE POINT CLOUD           │
└─────────────────────────────────────────────────┘
                        │
      ┌─────────────────▼───────────────────────────┐
      │   ┌─────────────────────────────────────────┐ │
      │   │      APPLY BEST MATCH TO THE LOADED MODEL│── 252
      │   │           TO FORM A NEW MODEL            │ │
      │   └─────────────────────────────────────────┘ │
      │                   │                            │
      │              254  ▼                            │
      │            ╱─────────────╲                     │
      │       Y   ╱    FURTHER    ╲                    │
      └──────────   INTERATION    ─                    │
                   ╲      ?       ╱
                    ╲─────────────╱
                         │ N
                         ▼
┌─────────────────────────────────────────────────┐
│ DETERMINE ROOTS OF A LINEAR SYSTEM OF EQUATIONS   │── 256
└─────────────────────────────────────────────────┘
                        │
                        ▼
┌─────────────────────────────────────────────────┐
│        SOLVE THE LINEAR SYSTEM OF EQUATIONS       │── 258
└─────────────────────────────────────────────────┘
                        │
                        ▼
                    ( END )
```

## FIG. 19

**FIG. 20**

*380*

**382** — RECEIVE RAW DATA SIGNAL DATA FROM ULTRASOUND MACHINE

--- TIER 1 PROCESSING ---

*384* SELECT FEATURE DETECTION MODEL

**386** — SELECT GAUSSIAN LINEAR/NON-LINEAR WITH LINEARIZATION MODEL

*410* — SELECT NON-LINEAR, NON-GAUSSIAN MODEL

**388** — ESTIMATE OPTIMAL TIME DELAY USING KALMAN FILTER TO IDENTIFY PEAKS (FIGS. 6A-6B)

*412* — ESTIMATE OPTIMAL TIME DELAY USING RECURSIVE BAYESIAN MODEL TO IDENTIFY PEAKS (FIGS. 7A-7D)

--- TIER 2 PROCESSING ---

*450* — APPLY BAYESIAN CORRELATION ESTIMATION AND SMOOTHING TO PEAKS IN TEMPORALLY ADJACENT SCAN LINES (FIG. 9)

*454* — EXTRACT FEATURES

--- TIER 3 PROCESSING ---

*462* — RECONSTRUCT 3-D SURFACE OF SCANNED FEATURE IN REAL TIME USING BAYESIAN INFERENCE MODEL

*460* — ULTRASOUND TRANSDUCER MOTION TRACKING DEVICE DATA

*464* — ESTIMATE POSITION AND SHAPE OF FEATURE THROUGH THE STATISTICAL PROPERTIES FROM SEQUENTIAL DATA OBTAINED FROM ULTRASOUND RF SIGNAL

# FIG. 21

**FIG. 22**

**FIG. 23A**

**FIG. 23B**

FIG. 24A

FIG. 24B

FIG. 24C

FIG. 24D

$448$

$442f$

$392f$

$446$

$442e$

$392e$

$S_{obs}$

$442d$

$392d$

$392c$

$442c$

$442b$

$392b$

$442a$

$392a$

$444a$

$444b$

$444c$

$444d$

$444e$

$444f$

$E(w_k^{i:1-N}, p_{est,fk+1}^{i:1-N})$

$\mathbb{P}(p_{6,f6}|s_{obs})$

$\mathbb{P}(p_{5,f5}|s_{obs})$

$\mathbb{P}(p_{4,f4}|s_{obs})$

$\mathbb{P}(p_{3,f3}|s_{obs})$

$\mathbb{P}(p_{2,f2}|s_{obs})$

$\mathbb{P}(p_{1,f1}|s_{obs})$

6

5

4

3

2

1

k

POSTERIOR DENSITY

## FIG. 25

$442$

DETECTED AS FIRST PEAKS

WITH BAYESIAN SMOOTHING

● ENVELOP PEAKS

$442$ $442$ $442$ $442$ $442$ $442$ $442$ $442$ $442$ $442$ $442$ $442$ $442$

$452$

$452$

$452$

$452$

$452$

$452$

$456$

$458$

## FIG. 26

54

FIG. 27

## REFERENCES CITED IN THE DESCRIPTION

**Patent documents cited in the description**

- WO 2012018851 A

**Non-patent literature cited in the description**

- **VARSLOT T et al.** Computer Simulation of Forward Wave Propagation in Soft Tissue. *IEEE Transactions on Ultrasonics, Ferroelectrics, and Frequency Control,* September 2005, vol. 52 (9), 1473-1482
- **CHEN Z et al.** Bayesian Filtering: From Kalman Filters to Particle Filters, and Beyond. *Statistics,* August 2011, 1-69, http://citeseerx.ist.psu.edu/viewdoc/download?doi=10.1.1.107.7415&rep=rep1&type =pdf

- **MAHFOUZ M et al.** Automatic Methods for Characterization of Sexual Dimorphism of Adult Femora: Distal Femur. *Computer Methods in Biomechanics and Biomedical Engineering,* 2007, vol. 10 (6